# EUROPEAN PATENT SPECIFICATION

(11) **EP 4 139 935 B1**
(45) Date of publication and mention of the grant of the patent: **11.02.2026**
(21) Application number: 21721412.1
(22) Date of filing: 16.04.2021
(51) Int. Cl.: G16H 40/63, G16H 50/20

(54) **AUTOMATED ASSESSMENT OF COGNITIVE AND SPEECH MOTOR IMPAIRMENT**
AUTOMATISIERTE BEWERTUNG VON KOGNITIVEN UND SPRACHLICHEN MOTORISCHEN STÖRUNGEN
ÉVALUATION AUTOMATISÉE DES TROUBLES COGNITIFS ET DE LA MOTRICITÉ DE LA PAROLE

(30) Priority: 22.04.2020 EP 20170921
(43) Date of publication of application: 01.03.2023
(73) Proprietor: F. Hoffmann-La Roche AG, 4070 Basel (CH)
(72) Inventor: LIPSMEIER, Florian, 4070 Basel (CH); STRAHM, Martin Christian, 4070 Basel (CH); WOLF, Detlef, 4070 Basel (CH); ZHANG SCHAERER, Yan-Ping, 4070 Basel (CH)
(74) Representative: Mewburn Ellis LLP
(86) International application number: PCT/EP2021/059972
(87) International publication number: WO 2021/213935

(56) References cited:
- US-A1- 2015 265 205

## Description

### Field of the Invention

The present invention relates to computer-implemented methods for automated assessment of cognitive impairment and/or speech motor impairment, comprising in particular analysing voice recordings from word-reading tests. Computing devices implementing the methods are also described. The methods and devices of the invention find applications in the clinical assessment of diseases and conditions that affect speech motor abilities and/or cognitive abilities, including neurological disorders such as Huntington's Disease.

### Background to the invention

The three-part Stroop test (word, color, and interference), originally proposed by Stroop in 1935 [3], is commonly used to monitor cognitive function. For example, it forms part of the cognitive test battery performed to quantify Huntington's Disease (HD) severity according to the widely used Unified Huntington's Disease Rating Scale (UHDRS). Huntington's disease (HD) is a neurodegenerative disease with progressive motor, cognitive and neuropsychiatric impairments [1]. The UHDRS assesses four domains: motor function, cognitive function, behavioral abnormalities, and functional capacity [2]. The word and color parts of the Stroop test represent the "consistent condition" in which color-words are printed in black ink and color-patches are printed in a matching ink color. In the interference part, color-words are printed inconsistently with ink color. Patients are required to read words or name ink colors as fast as possible. The clinician interprets the response as correct or incorrect. Scores are reported as the number of correct answers in each condition for a given 45-second period. The consistent condition is considered to measure processing speed and selective attention. The interference condition requires a mental conversion between words and colors, thus, is intended to measure cognitive flexibility.

Several studies investigated longitudinal change in cognitive function in Huntington's disease reached a similar conclusion that relatively automatic, speed-based tasks with low cognitive demands showed significant decline in early manifest HD patients [4-8]. In particular, the TRACK-HD cohort has enabled the discovery of a range of potential outcome measures for therapeutic trials in HD. Cognitive decline measured by the Stroop word-reading test showed great promise for clinical trial applications [5-7]. However, clinical assessment is often time-consuming and requires a certified neurologist or trained rater.

The use of speech analysis software to quantify speech rate in a reading task [20] and speech rhythm instability in a speaking task [12] have been suggested. Both of these measures were found to differ between HD patients and healthy subjects. However, these approaches have limited practical applicability, and in particular are not directly applicable to measure performance in tasks such as those in the Stroop test. Indeed, the quantification of speech rate in Skodda et al. [20] relies on the use of the oscillographic sound pressure signal to measure the length of syllables and pauses, while the approach in Rusz et al. [12] relies on automated detection of syllables in a repeated syllables speaking task (a single syllable is repeated at a "comfortable, self-determined, steady pace"). Both approaches are likely to lack sensitivity in the context of fast speech in the word part of the Stroop test. Further, neither of these approaches is able to assess the correctness of the word reading task in the Stroop test. Speech recognition software using deep learning models (e.g. Mozilla's free speech recognition project DeepSpeech) and hidden Markov models (e.g. Carnegie Mellon University's Sphinx toolkit) could in theory be used to understand the speech content in a word reading task. However, these approaches use pre-trained models that are built on healthy populations and are language dependent. As such, they are unlikely to be very accurate when applied to patients with speech impairments, and would have limited applicability for analysis of multi-language cohorts.

Therefore, there is still a need for improved methods to automatically assess speech and cognitive impairment using voice recordings.

US 2015/265205 A1 (Rosenbek John Clyde [US] et al) describes screening for neurological disease using speech articulation characteristics.

### Statements of invention

The claimed invention is defined by the appended claims.

The inventors have developed a new device and method for automated assessment of cognitive impairment, comprising in particular analysing voice recordings from word-reading tests. The method is model-free, language-independent and applicable to subjects with speech impairments. It enables a fully automated accurate assessment of number of correct words, thereby enabling self-assessment of disease symptoms, in particular cognitive function, remotely in large populations.

A first aspect of the present invention thus provides a method of assessing cognitive impairment in a subject, the method comprising obtaining a voice recording from a word-reading test from the subject and analysing the voice recording, or a portion thereof, by: identifying a plurality of segments of the voice recording that correspond to single words or syllables; and determining the number of correctly read words in the voice recording, wherein the voice recording is from a word-reading test comprising reading a sequence of words drawn from a set of n words. The method comprises: computing one or more Mel-frequency cepstral coefficients (MFCCs) for the segments, to obtain a plurality of vectors of values, each vector being associated with a segment; and clustering the plurality of vector of values into n clusters, wherein each cluster has *n* possible labels corresponding to each of the *n* words. The method further comprises, for each of the *n!* permutations of labels, predicting a sequence of words in the voice recording using the labels associated with the clustered vectors of values, and performing a sequence alignment between the predicted sequence of words and the sequence of words used in the word reading test; and selecting the labels that result in the best alignment and counting the number of matches in the alignment, wherein the number of matches corresponds to the number of correctly read words in the voice recording, and wherein the number of correctly read words is indicative of the level of cognitive impairment of the subject.

The method may further comprise determining the speech rate associated with the voice recording by counting the number of segments identified in the voice recording. Identifying segments of the voice recording that correspond to single words or syllables may comprise: obtaining a power Mel-spectrogram of the voice recording; computing the maximum intensity projection of the Mel spectrogram along the frequency axis; and defining a segment boundary as the time point where the maximum intensity projection of the Mel spectrogram along the frequency axis crosses a threshold.

Where the method comprises determining the number of correctly read words in the voice recording, the voice recording is from a word-reading test comprising reading a sequence of words drawn from a set *of n* words, and the method comprises: computing one or more Mel-frequency cepstral coefficients (MFCCs) for each the segments to obtain a plurality of vectors of values, each vector being associated with a segment; clustering the plurality of vector of values into *n* clusters, wherein each cluster has *n* possible labels corresponding to each of the *n* words; for each of the *n!* permutations of labels, predicting a sequence of words in the voice recording using the labels associated with the clustered vectors of values, and performing a sequence alignment between the predicted sequence of words and the sequence of words used in the word reading test; and selecting the labels that result in the best alignment and counting the number of matches in the alignment, wherein the number of matches corresponds to the number of correctly read words in the voice recording. Where the method comprises determining the speech rate associated with the voice recording by counting the number of segments identified in the voice recording, identifying segments of the voice recording that correspond to single words or syllables comprises: obtaining a power Mel-spectrogram of the voice recording; computing the maximum intensity projection of the Mel spectrogram along the frequency axis; and defining a segment boundary as the time point where the maximum intensity projection of the Mel spectrogram along the frequency axis crosses a threshold.

In embodiments, the voice recording is from a word-reading test comprising reading a sequence of words drawn from a set of *n* words, and the method may comprise identifying a plurality of segments of the voice recording that correspond to single words or syllables by: obtaining a power Mel-spectrogram of the voice recording; computing the maximum intensity projection of the Mel spectrogram along the frequency axis; and defining a segment boundary as the time point where the maximum intensity projection of the Mel spectrogram along the frequency axis crosses a threshold. The method further comprises determining the number of correctly read words in the voice recording by:computing one or more Mel-frequency cepstral coefficients (MFCCs) for the segments to obtain a plurality of vectors of values, each vector being associated with a segment; clustering the plurality of vector of values into *n* clusters, wherein each cluster has *n* possible labels corresponding to each of the *n* words; for each of the *n!* permutations of labels, predicting a sequence of words in the voice recording using the labels associated with the clustered vectors of values, and performing a sequence alignment between the predicted sequence of words and the sequence of words used in the word reading test; and selecting the labels that result in the best alignment and counting the number of matches in the alignment, wherein the number of matches corresponds to the number of correctly read words in the voice recording. Optionally, the method further comprises determining the speech rate associated with the voice recording by counting the number of segments identified in the voice recording.

The approach to word/syllable segmentation according to the present invention enables the accurate and sensitive segmentation of words (and in some cases also syllables from multi-syllables words) from voice recordings even where the speech is relatively fast paced (i.e. comprising no or short pauses between words), where existing methods typically based on energy envelopes may not perform well. It further enables the automated quantification of the speech rate in a word reading task from data that can be simply and easily acquired remotely, such as e.g. by a patient recording themselves reading words displayed on a computing device (e.g. a mobile computing device such as a smartphone or tablet or a personal computer, through an application or web application, as will be described further herein).

A segment of voice recording corresponding to a single word or syllable may be defined as a segment comprised between two consecutive word/syllable boundaries. Preferably, a segment of voice recording corresponding to a single word/syllable may be defined as a segment between a first boundary where the maximum intensity projection of the Mel spectrogram crosses the threshold from a lower to a higher value, and a second boundary where the maximum intensity projection of the Mel spectrogram crosses the threshold from a higher to a lower value. Advantageously, segments of voice recording between boundaries that do not satisfy this definition may be excluded.

Advantageously, the approach to correct word counting described herein is entirely driven by the data and is as such model and language independent. In particular, as the clustering step is an unsupervised learning step, it does not require any knowledge of the actual word that each group of segments represents (ground truth). In alternative embodiments, it is possible for the clustering to be replaced with a supervised learning approach such as a Hidden Markov Model. However, such an approach would require a re-training of the model for each language.

Advantageously, the approach to correct word counting described herein is further able to deal with speech impairments such as dysarthria which may prevent conventional word recognition approaches from identifying words that are correctly read but incorrectly pronounced, thereby representing a more faithful measure of cognitive impairment. It further enables the automated quantification of the number of correctly read words in a word reading task from data that can be simply and easily acquired remotely, such as e.g. by a patient recording themselves reading words displayed on a computing device (e.g. a mobile computing device such as a smartphone or tablet).

In other words, the correct word counting approach according to the present invention can be used alone or in combination with the speech rate according to the disclosure to monitor cognitive impairment in a patient remotely, with no manual input and in particular no clinically trained staff input.

Advantageously, the approach to correct word counting is performed in combination with the approach to word/syllable segmentation described above.. Alternatively, other word segmentation methods may be used, as known in the art.

In preferred embodiments of any aspect, the words are monosyllabic words or disyllabic words. It may further be advantageous for all of the words to have the same number of syllables. For example, it may be advantageous for all words to be either monosyllabic or disyllabic.

Embodiments using only monosyllabic words may be particularly advantageous because in such embodiments each segment corresponds to a single word. Such embodiments therefore advantageously result in counts of the number of segments that correspond to the number of words read and/or in the timing of segment being directly usable to obtain a speech rate (or any other feature associated with the rhythm of the speech). Further, the n words being monosyllabic may improve the accuracy of the clustering, as a single vector of values is expected for each word, resulting in n clusters that are expected to be relatively homogeneous. The use of monosyllabic words may also improve the accuracy of speech rate determination as it removes any potential problems that may be associated with identifying syllables that belong to the same word.

Embodiments using only disyllabic words may advantageously result in counts of the number of segments that can be related to the number of words read (and hence the speech rate) in a straightforward manner, and/or that can be compared across voice recordings from word-reading tests with the same characteristics.

In some embodiments using disyllabic words, the method may further comprise excluding segments that correspond to a specified one of two syllables in a word, prior to counting the number of segments identified in the voice recording and/or prior to determining the number of correctly read words in the voice recording. Segments that correspond to one of two syllables in a word may be identified based on the relative timing of two consecutive segments. For example, segments that closely follow each other such as e.g. segments that add up to less than a specific time (e.g. 400 ms), and/or that are separated by less than a specific time (e.g. 10 ms) may be assumed to belong to the same word. A specified segment to be excluded may further be identified as the first or second segment of two segments assumed to belong to the same word. Alternatively, a specified segment to be excluded may be identified based on the characteristics of the sound signal in the two segments.

For example, the segment with lowest energy may be excluded. As another alternative, a specified segment to be excluded may be identified based on the relative length of the two segments. For example, the segment with shortest length may be excluded. Alternatively, the method may comprise merging segments that correspond to a specified one of two syllables in a word with a segment that closely follows or precedes it, such as e.g. segments that are within a specified time (e.g. 10 ms) of each other. Without wishing to be bound by any particular theory, it is believed that merging segments corresponding to syllables of the same word may be particularly difficult when analysing fast speech. As such, merging segments that are within a specified time of each other is believed to be particularly suitable for speech that has a speed similar to free speech or lower. In embodiments where the speech is expected to be relatively fast, it may be advantageous to use segments that are assumed to correspond to single syllables directly, rather than merging or excluding segments.

In embodiments using disyllabic words (or multi-syllabic words in general), the disyllabic words preferably have one emphasized syllable. Without wishing to be bound by theory, it is believed that the clustering step may have increased robustness to the presence of "noise" coming from segments corresponding to syllables rather than words when one of the syllable is emphasized. Indeed, in such case the signal from a non-emphasized syllable may be considered as noise in the clustering process, which will still produce clusters that are homogeneous in terms of the identity of the emphasized syllables assigned to each cluster.

In embodiments, predicting a sequence of words in the voice recording using the labels associated with the clustered vectors of values comprises predicting a sequence of words that correspond to the respective cluster labels for each of the clustered vectors of values, ordered following the order of the segments from which the vectors of values were derived.

In some embodiments, predicting a sequence of words in the voice recording using the labels associated with the clustered vectors of values comprises predicting a sequence of words that correspond to the respective cluster labels for each of the clustered vectors of values that are assigned to a cluster with a confidence that meets one or more predetermined criteria. In other words, predicting a sequence of words in the voice recording using the labels associated with the clustered vectors of values comprises may comprise excluding predictions for clustered vectors of values that are not associated with any specific clusters with a confidence that meets one or more predetermined criteria. The one or more predetermined criteria may be defined using a threshold on the probability that a vector of values belong to one of the n clusters, the distance between a vector of values and a representative vector of values for one of the n clusters (e.g. the coordinates of the medoid or centroid of the cluster), or combinations thereof.

In some embodiments, predicting a sequence of words in the voice recording using the labels associated with the clustered vectors of values comprises predicting a sequence of words that correspond to the respective cluster labels for each of the clustered vectors of values. In some such embodiments, where multi-syllable words (especially multi-syllable words comprising one emphasized syllable) are used, multiple word predictions may be predicted for a multi-syllable word, since multiple segments may be identified and clustered. The present inventors have found that it would still be possible to determine the number of correctly read words in the voice recording according to the methods described herein in such situations. Indeed, as explained above, it is believed that the clustering step may be robust to the presence of "noise" coming from additional syllables, such that clusters primarily determined by single syllables in each of the n words could still be identified. Further, it is believed that the sequence alignment step would be able to deal with such additional syllables as insertions within the sequence, which insertions would be present for each of the *n!* permutations of labels since they result from the presence of additional predicted words that are not expected to be present in the sequence of words used in the word reading test. As such, the number of matches in the alignment would still correspond to the number of correctly read words in the voice recording.

Within the context of the present invention, the subject is a human subject. The words "subject", "patient" and "individual" are used interchangeably throughout this disclosure.

Within the context of the present invention, an individual may be considered to have a speech motor impairment if the individual's ability to speak is affected by neuromuscular, musculoskeletal or respiration impairment. Any condition, disease or disorder that affects an individual's ability to speak by affecting their ability to execute the motor movements (including oral and/or respiratory movements) necessary for the production of speech may cause a speech motor impairment. Examples of conditions, diseases or disorders that may cause a speech motor impairment include:
(i) neuromuscular diseases such as cerebrovascular accidents (strokes), Parkinson's disease (PD), multiple sclerosis (MS), Huntington's disease (HD), Creutzfeldt-Jakob disease, amyotrophic lateral sclerosis (ALS), progressive bulbar palsy (PBP), cerebral palsy (CP), muscular dystrophy (MD), developmental verbal dyspraxia;
(ii) physical damage to the head, neck, larynx or lungs; and
(iii) diseases, disorders or conditions that cause a shortness of breath, such as respiratory diseases or disorders (including obstructive lung disease (e.g. asthma, chronic bronchitis, bronchiectasis and chronic obstructive pulmonary disease (COPD)), chronic respiratory diseases (CRDs), respiratory tract infections, and lung tumours), obesity, heart failure, and panic attacks.

As such, the methods described herein may find uses in the diagnosing, monitoring or treatment of any of the above conditions, diseases or disorders.

Within the context of the present invention, an individual may be considered to have a cognitive impairment if the individual's cognitive abilities are affected. In particular, any condition, disease or disorder that affects an individual's ability to process information in a printed text that the individual is reading may be considered to cause cognitive impairment within the context of the present disclosure.

Examples of diseases, disorders or conditions that may affect an individual's ability to process information in a printed text that the individual is reading include:
(i) dementia such as dementia caused by strokes, Parkinson's disease (PD), Huntington's disease (HD), Creutzfeldt-Jakob disease,
(ii) neuropsychological deficits such as attention deficit hyperactivity disorder (ADHD), neuropsychological deficits caused by extreme tiredness (such as e.g. caused by heart failure) or old age, and
(iii) brain injuries and tumours.

As such, the methods described herein may find uses in the diagnosing, monitoring or treatment of any of the above conditions, diseases or disorders.

Within the context of the present invention, a word-reading test (also referred to herein as a "word reading task") refers to a test that requires an individual to read a set of words that are not connected so as to form a sentence (e.g. the words may be drawn randomly or pseudo-randomly from a set). For example, all of the words in the set of words may be nouns, such as the words for a set of colours in a chosen language.

In embodiments, identifying segments of the voice recording that correspond to single words or syllables further comprises normalising the power Mel-spectrogram of the voice recording. Preferably, the power Mel-spectrogram is normalised against the frame that has the highest energy in the recording. In other words, each value of the power Mel-spectrogram may be divided by the highest energy value in the power Mel-spectrogram.

As the skilled person understands, a power Mel-spectrogram refers to a power spectrogram for a sound signal, on a Mel scale. Further, obtaining a Mel-spectrogram comprises defining frames along the voice recording (where a frame can correspond to the signal in a window of fixed width applied along the time axis) and computing a power spectrum on a Mel scale for each frame. This process results in a matrix of values of power per Mel unit per frame (time bin).

As the skilled person understand, obtaining the maximum intensity projection against the frequency axis for such a spectrogram comprises selecting the maximum intensity on the Mel spectrum for each frame.

The normalisation advantageously eases the comparison between different voice recordings, which may be associated with the same or different subjects. This may be particularly advantageous e.g. where multiple individual recordings from the same subject are combined. For example, this may be particularly advantageous where short recordings are preferred (e.g. because the subject is frail), where a standard or otherwise desirable length of word reading test is preferable.. Normalising the Mel-spectrogram against the frame that has the highest energy in the recording advantageously results in the loudest frame in the recording having a relative energy value (value after the maximum intensity projection) of 0dB for any recording. Other frames will have relative energy values below 0 dB. Further, as normalising the power Mel-spectrogram results in maximum intensity projections that represent relative energy (values of dB over time) comparable between voice recordings, a common threshold (which may be predetermined or dynamically determined) can advantageously be used for multiple recording.

In embodiments, the threshold is dynamically determined for each recording. Preferably, the threshold is determined as a function of the maximum intensity projection values for the recording. For example, the threshold may be determined as a weighted average of the relative energy values assumed to correspond to signal and the relative energy values assumed to correspond to background noise. The relative energy values assumed to correspond to signal may be e.g. the top x (where x can be e.g. 10%) relative energy values observed in the recording. Similarly, the relative energy values assumed to correspond to background noise may be e.g. the bottom x (where x can be e.g. 10%) relative energy values observed in the recording. The present inventors have found the use of the average value of the top 10% relative energy values across frames and the average value of the bottom 10% relative energy values across frames to be particularly convenient. Alternatively, a predetermined value of relative energy assumed to correspond to signal (i.e. voice signal) may be used. For example, a value of about -10 dB has been commonly observed by the inventors and could be usefully chosen. Similarly, a predetermined value of relative energy assumed to correspond to background noise (may be used. For example, a value of about -60 dB has been commonly observed by the inventors and could be usefully chosen.

Where the threshold is determined as a weighted average of the relative energy values assumed to correspond to signal and the relative energy values assumed to correspond to background noise, the weight for the latter may be chosen between 0.5 and 0.9, and the weights for the former may be chosen between 0.5 and 0.1. In embodiments, the weight for the background noise contribution may be higher than the weight for the signal contribution. This may be particularly advantageous when the voice recording has been pre-processed by performing one or more noise-cancelling steps. Indeed, in such cases the bottom part of the signal (low relative energies) may contain more information than expected for a signal that has not been pre-processed for noise cancelling. Without wishing to be bound by theory, the present inventors have observed that many modern mobile device produce voice recordings that are already pre-processed to some extent in this way. As such, the present inventors have found it useful to emphasise the bottom end of the relative energy values to some extent. The present inventors have found weights of about 0.2 and about 0.8, respectively for the signal and background noise contributions, to be advantageous. As the skilled person understands, the exact thresholds that are advantageous may be determined by trial-and-error and/or formal training using training data.

In embodiments, the threshold is predetermined. In embodiments, the predetermined threshold is chosen between -60 dB and -40 dB, such as e.g. -60 dB, -55 dB, -50 dB, -45 dB, or -40 dB. Preferably, the predetermined threshold is about -50 dB. The inventors have found that this threshold stroke a good balance between sensitivity and specificity of word/syllable boundary identification in good quality voice recordings, and particularly in voice recordings that have been pre-processed using one or more noise cancellation steps.

Without wishing to be bound by theory, it is believed that the use of a dynamically determined threshold may be particularly advantageous where the voice recording comprises a reference tone and/or where the signal-to-noise ratio is good (e.g. above a predetermined threshold, such as 30 dB). Conversely, the use of a predetermined threshold may be particularly advantageous where the voice recording does not comprise a reference tone and/or the signal-to-noise ratio is poor.

In embodiments, determining the speech rate associated with the voice recording comprises computing a cumulative sum of the number of identified segments in the voice recording over time, and computing the slope of a linear regression model fitted to the cumulative sum data. In embodiments, determining the speech rate associated with the voice recording comprises computing a rate of speech timing (RST) as described in Hlavnicka, J., et al., 2017 [19].

Advantageously, this approach results in a robust estimate of the speech rate as a number of words per unit of time over the entire recording. The estimate thus obtained may be robust to outliers (such as e.g. distractions that may cause isolated momentary changes of the speech rate), while being sensitive to genuine slowing of the speech rate (such as e.g. where genuine speech motor function or cognitive impairment leads to frequent segments with slow speech). Additionally, this approach is independent of the length of the recording. As such, it may enable the comparison of the speech rates obtained for voice recordings of different lengths, or for different portions of the same voice recording. Further, it may be robust to external factors such as a subject pausing or not speaking for reasons not related to cognitive / speech motor impairment (such as e.g. because the subject forgets to restart from the top of the list of words if they have read the entire list, or initially does not realise that the recording has started). Further, this approach is also advantageously robust to uncertainty in relation to the specific timing of the start of words and/or to variation in the duration of the words be taken into account.

In other embodiments, determining the speech rate associated with the voice recording comprises computing the total number of words in the recording and diving the total number of words by the length of the recording. The total number of words may be assumed to be equal to the total number of segments, or may be derived from the total number of segments, for example by excluding segments as described herein.

In yet other embodiments, determining the speech rate associated with the voice recording comprises dividing the recording into multiple equal time bins, computing the total number of words in each time bin, and computing a summarised measure of the speech rate across time bins. For example, the average, trimmed average or median speech rate across time bins may be used as a summarised measure of the speech rate. The use of the median or trimmed average may advantageously reduce the effect of outliers such as e.g. bins that do not contain any words. The number of words in a time bin may be assumed to be equal to the number of segments in the bin, or may be derived from the number of segments in the bin, for example by excluding segments as described herein.

In embodiments, obtaining a power Mel-spectrogram of the voice recording comprises applying a sliding window (preferably with a size of 15 ms and a step size of 10 ms) and 138 triangular filters spanning the range of 25.5 Hz to 8 kHz. Without wishing to be bound by theory, it is believed that using relatively narrow time windows (e.g. 10-15 ms, as opposed to e.g. 25 ms and above) may be useful in the context of identifying segments that correspond to single words or syllables, and in particular for the purpose of identifying segment boundaries that correspond to the start of words or syllables. This is because using relatively narrow time windows may increase the sensitivity of the detection, whereas wider time windows may smooth out small signals that may be informative.

As the skilled person understand, the overlapping triangular filters (typically 138) applied to a frequency spectrogram (Hz scale) are commonly used to obtain a spectrogram in Mel scale. Further, spanning the range of 25.5 Hz to 8 kHz has been found to be advantageous as this adequately captures the human hearing range.

In embodiments, identifying segments of the voice recording that correspond to single words or syllables further comprises: performing onset detection for at least one of the segments by computing a spectral flux function over the Mel-spectrogram for the segment; and defining a further boundary whenever an onset is detected within a segment, thereby forming two new segments.

Onset detection using a spectral flux function is commonly used for the analysis of music recordings, for beat detection. As the skilled person understands, onset detection using the spectral flux function is a method that looks at the derivative of the energy signal. In other words, the spectral flux function measures how quickly the power spectrum of the signal is changing. As such, it may be particularly useful to identify "valleys" in the signal (sudden changes in the energy signal) that may correspond to the start of new words or syllables within a segment. This may advantageously "refine" the segmentation where necessary. Without wishing to be bound by theory, the present inventors believe that this approach is particularly useful as a "refinement step" where words/syllable boundaries have already been identified using a less sensitive approach resulting in "coarse" segments. This is at least in part because the approach can be applied independently to a segment, with appropriate parameters (e.g. threshold for onset detection) for the segment,

Preferably, performing onset detection comprises computing an onset strength (based on the spectral flux function but including a spectral-trajectory tracking stage to the common spectral flux calculation method) over time from the power Mel-spectrogram, using the superflux method described in Böck S and Widmer G [13]. In embodiments, performing onset detection comprises computing the onset strength function over time from the power Mel-spectrogram, using the superflux method as implemented in the LibROSA library (https://librosa.github.io/librosa/, see function *librosa.onset.onset_strength;* McFee et al. [21]).

Preferably, performing onset detection further comprises normalising the onset strength function for the segment to a value between 0 and 1. This may be achieved for example by dividing each value of the onset strength function by the maximum onset strength within the segment. Normalising the onset strength function may result on a reduction of the number of false positive detections.

In embodiments, performing onset detection comprises applying a threshold to the spectral flux function or a function derived therefrom, wherein an onset is detected where the function increases above the threshold. In embodiments, performing onset detection comprises normalising the onset strength function for the segment to a value between 0 and 1 and separating segments into sub-segments if the normalised onset strength is above a threshold. The present inventors have found a threshold of between 0.1 and 0.4, preferably between 0.2 and 0.3 to result in particularly low rates of false positives when applied to the normalised onset strength function. An appropriate threshold may be defined as a threshold that minimises the rate of false positive detections when the method is applied to training data.

In embodiments, performing onset detection further comprises smoothing the (optionally normalised) onset strength function for the segment. For example, smoothing may be obtained by calculating a moving average with a fixed window size. For example, a window size of 10-15 ms, such as e.g. 11 ms may be useful. Smoothing may further reduce the rate of false positives detected.

Preferably, identifying segments of the voice recording that correspond to single words or syllables further comprises performing onset detection for each of the segments and defining a further boundary whenever an onset is detected within a segment, thereby forming two new segments.

In embodiments, identifying segments of the voice recording that correspond to single words or syllables further comprises excluding segments that represent erroneous detections by computing one or more Mel-frequency cepstral coefficients (MFCCs) for the segments to obtain a plurality of vectors of values, each vector being associated with a segment, and applying an outlier detection method to the plurality of vectors of values.

The present inventors have found that applying an outlier detection method to data derived from single word/syllable segments advantageously enables the removal of segments that correspond to erroneous detections (such as e.g. those caused by imprecise articulation, respirations and non-speech sound.)

In embodiments, identifying segments of the voice recording that correspond to single words/syllables further comprises excluding segments that represent erroneous detections by removing segments shorter than a predetermined threshold and/or with mean relative energy below a predetermined threshold. For example, segments shorter than 100 ms may advantageously be excluded. Similarly, segments with a mean relative energy below -40 dB may advantageously be excluded. The present inventors have found that such an approach simply and efficiently excluded segments that did correspond to words or syllables. Preferably, the segments are filtered to exclude short and/or low energy segments prior to calculating MFCCs for segments and applying an outlier detection method as explained above. Indeed, this advantageously avoids the unnecessary step of computing MFCCs for erroneous segments, and prevents such erroneous segments from introducing further noise in the outlier detection method.

As the skilled person understands, computing one or more Mel-frequency cepstral coefficients (MFCCs) for a segment typically comprises defining frames along the segment of voice recording (where a frame can correspond to the signal in a window of fixed width applied along the time axis). The window is typically a sliding window, i.e. a window of set length (e.g. 10-25 ms, such as 25 ms) moved along the time axis with a defined step length (e.g. 3-10 ms, such as 10 ms), resulting in frames that are partially overlapping. Computing one or more MFCCs typically further comprises, for each frame, computing a Fourier transform (FT) of the signal within the frame, mapping the powers of the spectrum thus obtained onto the Mel scale (e.g. using triangular overlapping filters), taking the log of the powers at each of the Mel frequencies and performing a discrete cosine transform of the signal thus obtained (i.e. obtaining a spectrum-of-spectrum). The amplitudes of the resulting spectrum represent the MFCCs for the frame. As explained above, a set of 138 Mel values is commonly obtained for the power Mel-spectrum (i.e. the frequency range is commonly mapped to 138 Mel scale values using 138 overlapping triangular filters). However, through the process of calculating MFCCs, this information is compressed into a smaller set of values (the MFCCs), typically 13 values. In many cases, the information contained in multiple of the 138 Mel values will be correlated such that compression of this signal does not result in a detrimental loss of informative signal.

In particular, computing one or more Mel-frequency cepstral coefficients (MFCCs) for a segment may be performed as described in Rusz et al. [16]. Computing one or more Mel-frequency cepstral coefficients (MFCCs) for a segment may be performed as implemented in the LibROSA library (https://librosa.github.io/librosa/; McFee et al. [21]; see *librosa.feature.mfcc*)*.* Alternatively, computing one or more MFCCs for a segment may be performed as implemented in the library "python_speech_features" (James Lyons et al., 2020) [22].

In embodiments, computing one or more Mel-frequency cepstral coefficients (MFCCs) for a segment comprises computing at least the first 3 MFCCs (optionally, all 13 MFCCs) for each frame of the segment and computing a summarised measured for each MFCC across frames in the segment, thereby obtaining a vector of at least 3 values (one for each MFCC used) for the segment.

In embodiments, the number and/or identity of the at least 3 MFCCs used for the outlier detection method may be determined using training data and/or internal control data. For example, the at least 3 MFCCs may be chosen as the minimal set of MFCCs that is sufficient to remove a percentage (e.g. at least 90%, or at least 95%) of erroneous detections in training data. As another example, the at least 3 MFCCs may be chosen as the minimal set of MFCCs that is sufficient to remove a segment corresponding to an internal control (such as e.g. a reference tone as will be described further below).

Preferably, only the first 3 MFCCs are used for the outlier detection method. This advantageously captures the information that enables the separation of true words/syllables from erroneous detections (e.g. respiration, non-speech sound), without introducing information that could result in different words forming separate distributions of points that could confuse the outlier detection process.

In embodiments, applying an outlier detection method to the plurality of vectors of values comprises excluding all segments whose vector of values is above a predetermined distance from the remaining vectors of values.

Preferably, the distance between a specific vector of values and the remaining vectors of values can be quantified using the Mahalanobis distance. The Mahalanobis distance is a convenient measure of the distance between a point and a distribution. It has the advantage of being unitless, scale-invariant, and taking into account the correlations in the data. Alternatively, the distance between a specific vector of values and the remaining vectors of values can be quantified using the distance (e.g. Euclidian distance, Manhattan distance) between the specific vector of values and a representative value (e.g. the mean or medoid) for the remaining vectors of values. The values may optionally be scaled, such as e.g. to have unit variance along each coordinate, prior to applying outlier detection.

In embodiments, the predetermined distance is chosen depending on the observed variability in the plurality of vectors of value. For example, the predetermined distance may be a multiple of a measure of variability in the data, such as the standard deviation, or the value of a chosen quantile. In such embodiments, the predetermined distance may be chosen depending on the expected proportion of erroneous detections. The present inventors have found that a threshold of between 1 and 3 standard deviations around the mean of the plurality of vectors of values enabled accurate removal of outliers. A threshold of 2 standard deviations was found advantageous, particularly when an expected proportion of erroneous detections is around 5 %.

An arguably similar approach to erroneous detection removal is described in Rusz et al. [16]. However, the approach described in this document is significantly more complex than that of the present disclosure. In particular, it relies on an iterative process where at each iteration inliers and outliers are identified using a quantile-based threshold on the distribution of mutual distances, then outliers are excluded using a quantile-based threshold on the distribution of distances between inliers and outliers as previously defined.

The present inventors have found that a simpler approach as described herein was advantageous in the context of the present invention. Without wishing to be bound by theory, the approach to erroneous detection removal described herein is believed to be particularly advantageous in the present context because the proportion of erroneous detection is low. This may be due in part to the segment detection approach described herein having very high accuracy. Without wishing to be bound by theory, the present inventors believe that the approach to syllable segmentation used in Rusz et al. [16] (which relies on parametrizing the signal to 12 MFCCs inside a sliding window of 10 ms length-3ms step, searching for a low frequency spectral envelope which can be described using the first 3 MFCCs, then computing the mean of each of the 3MFCCs inside each envelope and using k-means to separate these points into syllables and pauses) is not as accurate as that of the present invention. This at least in part because it is designed to identify a contrast between pauses and words, where the words are all identical, and partially because the approach in Rusz et al. [16] heavily relies on the iterative outlier detection process to increase the overall accuracy of the true positive segment identification process. Indeed, the Rusz et al. [16] approach was developed specifically to deal with syllable detection using voice recordings where the patient is asked to repeat the same syllable at a comfortable pace. As such, the data consists of only two expected categories of segments (pauses and syllables) of homogeneous content. In such cases using the first 3 MFCCs in combination with a complex iterative error detection process for segment identification may achieve good accuracy. However, this would have low accuracy in the context of analysing a voice recording from a word-reading test, at least because more than one type of syllables are expected. As such, this would also not be suitable for the purpose of obtaining a clinically informative correct number count.

Without wishing to be bound by theory, the present inventors further believe that the process of obtaining a correct number count as described herein is advantageously tolerant to the presence of erroneously detected segments at least to some extent. This is at least in part because the alignment step can include gaps for erroneous detections without significantly impacting the overall accuracy of the method.

Alternatively, other outlier detection methods that are applicable to a set of multidimensional observations may be used. For example, clustering approaches may be used.

In some embodiment of any aspect, the voice recording includes a reference tone. For example, the recording may have been obtained using a computing device configured to emit a reference tone shortly after starting to record the user performing the reading test. This may be useful to provide the user with an indication of when to begin the reading task.

In embodiments where the voice recording includes a reference tone, one or more parameters of the method may be chosen such that the reference tone is identified as a segment that corresponds to a single words or syllable, and/or such that a segment comprising the reference tone is excluded in a process to remove erroneous detections. For example, the set of MFCCs used in the erroneous detection removal process and/or the predetermined distance used in this process may be chosen such that the segment corresponding to the reference tone is removed in each voice recording (or at least a chosen percentage of voice recordings).

In embodiments, the words are colour words. In some such embodiments, the words are displayed in a single colour in the word reading test. In such a set-up, the number of correctly read words advantageously matches the Stroop word count from the first part (in the "consistent condition") of a three-part Stroop test. This measure is a commonly used clinical measure that may be used for example as part of the standard assessment of cognitive impairment in HD.

In embodiments, the words are colour words displayed in a colour that is not necessarily consistent with the meaning of the single words. For example, the words may be drawn randomly or pseudo-randomly from a set of colour words, and may each be displayed in a colour that is randomly or pseudo-randomly drawn from a set of colours. In embodiments, the words are colour words displayed in a colour that is inconsistent with the meaning of the single words. For example, the words may be drawn randomly or pseudo-randomly from a set of colour words, and may each be displayed in a colour that is randomly or pseudo-randomly drawn from a set of colours that excludes the colour that matches the colour word to be displayed. The colours in the set of colours for display may be identical or different from the colours in the set of colour words. In such embodiments, the number of correctly read words advantageously matches the Stroop word count from the third part ("inconsistent condition") of a three-part Stroop test. This measure is a commonly used clinical measure that may be used for example as part of the standard assessment of cognitive impairment in HD.

In embodiments, the words are drawn from a (closed) set of n words. In embodiments, n is between 2 and 10, preferably between 2 and 5, such as 3. The number n of different words in the set of words is preferably at least 2 because otherwise no reading is required after the subject has read the first word. The number n of different words in the set of words is preferably 10 or under 10 because otherwise the number of time that each word is expected to appear in a voice recording may be so low as to negatively impact the accuracy of the clustering process. Preferably, the number *n* of different words is chosen such that the number of times that each word is expected to appear in a voice recording is at least 10. As the skilled person understands, this may depend at least on the length of the voice recording and on the expected level of cognitive and/or speech motor impairment of the subject. A suitable choice for the number *n* of different words may for example be obtained using a comparable training cohort.

The n words may be colour words, such as e.g. the words for the colours "red", "green" and "blue" (i.e. in English: ['RED', 'GREEN', 'BLUE'], in German: ['ROT', 'GRÜN', 'BLAU'],in Spanish: ['ROJO', 'VERDE', 'AZUL'], in French: ['ROUGE', 'VERT', 'BLEU'], in Danish: ['RØD', 'GRØN', 'BLÅ'], in Polish: ['CZERWONY','ZIELONY', 'NIEBIESKI'], in Russian: [' 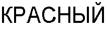 ', ' 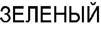 ', ' 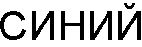 '], in Japanese: [' 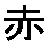 ', ' 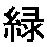 ', ' 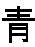 '], in Italian: ['ROSSO', 'VERDE', 'BLU'], in Dutch ['ROOD', 'GROEN', 'BLAUW'], etc). Colour words are commonly used in the word reading part of a Stroop reading test. The words for the colours "red", "green" and "blue" are common choices for this test and as such may advantageously enable the results of the test to be compared to or integrated with existing implementations of the Stroop test in a clinical context.

In embodiments, computing one or more MFCCs to obtain a vector of values for a segment comprises: computing a set of *i* MFCCs for each frame of the segment and obtaining a set of *j* values for the segment by compressing the signal formed by each of the *i* MFCCs across the frames in the segment, to obtain a vector of *ixj* values for the segment. For example, compressing the signal formed by each of the i MFCCs across the frames in the segment may comprise performing linear interpolation of said signal.

In embodiments, computing one or more MFCCs to obtain a vector of values for a segment comprises: computing a set of *i* MFCCs for each frame of the segment and obtaining a set of *j* values for the segment for each *i* by interpolation, preferably linear interpolation, to obtain a vector of *ixj* values for the segment.

As a result, the vector of values for each of the plurality of segments all have the same length. Such vectors of values can advantageously be used as an input for any clustering approach that identifies clusters of points in a multidimensional space.

Computing one or more MFCCs to obtain a vector of values for a segment may be performed as explained above. As the skilled person understand, the used of a fixed length time window to obtain MFCCs for a segment means that the total number of MFCCs per segment may vary depending on the length of the segment. In other words, a segment will have a number of frames *f,* each being associated with a set of *i* MFCCs, where *f* varies depending on the length of the segment. As a result, segments corresponding to longer syllables/words will be associated with a larger number of values than segments corresponding to shorter syllables/words. When these values are used as features representative of a segment for the purpose of clustering segments in a common space, this may be problematic. The interpolation step solves this problem. In embodiments, computing one or more MFCCs for a segment comprises computing a plurality of the second to thirteenth MFCCs for each frame of the segment. The first MFCCs is preferably not included. Without wishing to be bound by theory, it is assumed that the first MFCC represents the energy in a segment, which is primarily relevant to the recording condition and contains little information in relation to the identity of a word or syllable. By contrast, the remaining 12 MFCCs cover the human hearing range (by definition of MFCCs) and hence capture the sound features that relate to how humans produce and hear words.

In embodiments, the plurality of the second to thirteenth MFCCs comprise at least 2, at least 4, at least 6, at least 8, at least 10 or all 12 of the second to thirteenth MFCCs.

The present inventors have found that the second to thirteenths MFCCs contained information that could be used to distinguish words from a closed set of words as points in a hyperspace, using simple clustering approaches. In particular, as explained above, the second to thirteenth MFCCs cover the human hearing range and are therefore believed to capture the sound features that relate to how humans produce and hear words. As such, using those 12 MFCCs may advantageously capture the information that is believed to be relevant in differentiating one word/syllable from another in a human voice recording.

Where the segmentation method described herein is used, the MFCCs for each frame of the identified segments may already have been computed as part of the step of excluding segments that represent erroneous detections. In such embodiments, the previously computed MFCCs may advantageously be used to obtain a vector of values for the purpose of determining the number of correctly read words in the voice recording.

In embodiments, the parameter *j* is chosen such that *j* ≤ *f* for all segments used in the clustering step. In other words, the parameter *j* may be chosen such that the interpolation results in a compression of the signal (where for each MFCC, the signal is the value of said MFCC across the frames of the segment). In embodiments, the parameter *j* may be chosen such that the interpolation results in a compression of the signal by between 40 and 60% for all segments (or at least a set proportion, such as e.g. 90%, of the segments) used in the clustering. As the skilled person understands, using a fixed parameter *j*, the level of compression applied to a segment may depend on the length of the segment. Using a compression to between 40 and 60% of the signal may ensure that the signal in each segment is compressed to about half of its original signal density.

In convenient embodiments, *j* is chosen between 10 and 15, such as e.g. 12. Without wishing to be bound by theory, frames of 25 ms with a step size of 10 ms are commonly used for the calculation of MFCCs for a sound signal. Further, syllables (and monosyllabic words) may be about 250 ms long on average. As such, using *j= 12* may result in a compression from 25 values (corresponding to 25 frames over a 250 ms segment) on average, to about half of this number of values (i.e. about 40 to 60% compression on average).

In embodiments, clustering the plurality of vector of values into n clusters is performed using k-means. Advantageously, k-means is a simple and computationally efficient approach that was found by the inventors to perform well at separating words represented by vectors of MFCC values.

Further, the centroid of clusters obtained may correspond to a representation of the corresponding word or syllable in the MFCC space. This may provide useful information about the process (e.g. whether the segmentation and/or clustering has performed satisfactorily) and/or about the voice recording (and hence the subject). In particular, the centroid of such clusters can be compared between individuals and/or used as a further clinically informative measure (e.g. because it captures aspects of the subject's ability to articulate a syllable or word).

In embodiments, the one or more MFCCs are normalised across segments in a recording, prior to clustering and/or interpolation. In particular, each MFCC may be individually centred and standardised, resulting in each MFCC distribution having equal variance and a mean of zero. This may advantageously improve the performance of the clustering process, as it may prevent some MFCCs from "dominating" the clustering if they are distributed with high variance. In other words, this may ensure that all features in the clustering (i.e. each MFCC used) has a similar importance in the clustering.

Alternatively, other clustering approaches may be used such as partition around medoid or hierarchical clustering may be used.

In embodiments, performing a sequence alignment comprises obtaining an alignment score. In some such embodiments, the best alignment is the alignment that satisfies one or more predetermined criteria, at least one of these criteria applying to the alignment score. In embodiments, the best alignment is the alignment that has the highest alignment score.

In embodiments, the sequence alignment step is performed using a local sequence alignment algorithm, preferably the Smith-Waterman algorithm.

A local sequence alignment algorithm is ideally suited to the task of aligning two strings of letters selected from a closed set, where the strings are relatively short and may not necessarily have the same length (as is the case here since words may have been missed in the reading task and/or in the word segmentation process). In other words, a local sequence alignment algorithm such as the Smith-Waterman algorithm is particularly well-suited for the alignment of partially overlapping sequences, This is advantageous in the context of the present invention since alignments with mismatches and gaps are expected due to the subject achieving less than 100% correct words count and/or due to errors in the segmentation process.

In embodiments, the Smith-Waterman algorithm is used with a gap cost between 1 and 2 (preferably 2) and a match score = 3. The present inventors have found that these parameters led to an accurate identification of words in the voice recording, by comparison to manually annotated data.

Without wishing to be bound by theory, using a higher gap cost (e.g. 2 instead of 1) may lead to a restriction of the search space, and shorter alignments. This may advantageously capture a situation where matches are expected (i.e. it is assumed that there exists a cluster label assignment that is such that many characters of the predicted sequence of words can be aligned with characters of the known sequence of words).

There is provided a method of assessing cognitive impairment and optinally speech motor impairment in a subject, the method comprising analysing a voice recording from a word-reading test from the subject, or a portion thereof, using the method of any of the embodiments of the first aspect.

In embodiments, assessing cognitive impairment comprises measuring the number of correct words in the voice recording according to any of the embodiments of the first aspect. In embodiments, assessing cognitive impairment further comprises measuring the speech rate in the voice recording.

In embodiments, assessing speech motor impairment comprises measuring the speech rate in the voice recording. In embodiments, assessing speech motor impairment further comprises measuring the correct word count in the voice recording according to any of the embodiments of the first aspect.

Without wishing to be bound by theory, the present inventors have found that the speech rate and correct number count determined according to the present disclosure may provide complementary and correlated information about the level of cognitive and speech motor impairment in a subject.

Described herein is a method of assessing the severity of a disease, disorder or condition in a subject, the method comprising analysing a voice recording from a word-reading test from the subject, or a portion thereof, using the method of any preceding aspect, wherein the disease, disorder or condition is one that affects speech motor and/or cognitive abilities. Analysing a voice recording from a word-reading test from the subject may comprise determining a speech rate and/or a correct number count as described herein.

Described herein is a method of assessing the severity of symptoms in a subject having a disease, disorder or condition that affects speech motor and/or cognitive abilities, the method comprising analysing a voice recording from a word-reading test from the subject, or a portion thereof, using the method of any preceding aspect. Analysing a voice recording from a word-reading test from the subject may comprise determining a speech rate and/or a correct number count as described herein.

In embodiments, the subject is a subject with a neurological disease or disorder. For example, the subject may be a subject who has been diagnosed as having, is suspected of having or being likely to develop a neurological disease or disorder.

A neurological disease or disorder is a condition that affects the central and/or peripheral nervous system. The present invention is particularly advantageous in the context of neurological diseases or disorders that are expected to affect cognitive function and/or speech motor function. These may include Huntigton's Disease (HD), Parkinson's disease (PD), Alzheimer's disease (AD) and Attention deficit hyperactivity disorder (ADHD). In specific embodiments, the neurological disease or disorder is HD.

In embodiments, the subject is a subject who has been diagnosed as having or suspected of having a heart failure. The present inventors have found that the methods described herein could be used to assess a subject with heart failure, and in particular to assess the severity of symptoms such as shortness of breath and/or tiredness in such subjects.

As the skilled person understands, the method of analysing a voice-recording from a subject is a computer-implemented method. Indeed, analysis of voice recordings by syllable detection, classification and alignment as described require the analysis of large amounts of data through complex mathematical operations that are beyond the reach of mental activity.

In embodiments, the methods described herein are performed as part of clinical assessment of a disease, disorder or condition in a clinical trial. In embodiments, the methods are performed as part of clinical assessment of a neurological disease or disorder in a clinical trial.

In embodiments, the disease is HD. In such embodiments, the method may be performed as part of the Unified Huntington's Disease Rating Scale (UHDRS) assessment.

In embodiments, the method of any aspect further comprises obtaining a voice recording from a word-reading test from the subject.

In embodiments, obtaining a voice recording comprises receiving a word recording from a computing device associated with the subject. Optionally, obtaining a voice recording may further comprise causing a computing device associated with the subject to display a set of words and to record a voice recording.

Advantageously in such embodiments the steps of displaying a set of words for the word reading test, and recording the word recording can be performed by a computing device that is remote from a computing device that performs the analysis step. For example, the step of displaying and recording may be performed by a user's personal computing device (which may be a PC or a mobile device such as a mobile phone or tablet), while the analysis of the voice recording may be performed by a remote computer, such as a server. This may enable the remote acquisition of the clinically relevant data e.g. at a patients' home while leveraging increased computing capabilities are a remote computer, for analysis.

In embodiments, the computing device associated with the subject is a mobile computing device, such as a mobile phone or tablet. In embodiments, the step of causing a computing device associated with the subject to display a set of words and to record a voice recording is performed through an application, which may be a software application that runs locally on the computing device associated with the subject (sometimes referred to as "mobile app" or "native app" in the context of mobile devices), a web application that runs in a web browser, or a hybrid application that embeds a mobile website inside a native app.

In embodiments, the set of words comprises at least 20, at least 30, at least 40, at least 50 or about 60 words. In embodiments, the set of words are drawn at random from a set *of n* words. In embodiments, the method comprises drawing a set of words randomly from a set *of n* words and causing a computing device associated with the subject to display the set of words. In embodiments, the set of words are displayed in groups of *m* words on a line, where *m* can be e.g. 4. The display of 4 words per line was found herein to be convenient in the context of display on a typical smartphone screen. As the skilled person understand, the number of words (*m*) that is displayed as a group may be adjusted depending on the size of the screen/window on/in which the words are displayed and/or depending on preferences of the user (such as e.g. preferred font size). Such an adjustment may be automatic, for example through automatic detection of the screen or window size. Preferably, the groups of *m* words are displayed concomitantly. For example, all of the words in a line of e.g. 4 words are preferably displayed at the same time. This may reduce the risk that the results of the test are influenced by external parameters (i.e. parameters that are not indicative of a user's ability to perform a word reading test) such as e.g. lag in the display of successive words. In embodiments, a portion of the n words may be displayed concomitantly, which portion may be updated as the user progresses through the test, such as e.g. through the individual scrolling down. In embodiments, all of the *n* words are displayed concomitantly. Such embodiments may advantageously reduce the impact of external parameters such as e.g. lag in the display of successive words, delay in a user scrolling down or up to make new words appear or restart from the beginning of a set of words, etc.

In embodiments, the method comprises causing the computing device associated with the subject to record a voice recording of a set length of time. In embodiments, the method comprise causing the computing device to emit a fixed length tone, then to record a voice recording. A set length of time is advantageously at least 30 seconds. A length of time is preferably chosen such that the voice recording can be expected to contain a sufficient number of words for reliable speech rate and/or correct number counts to be obtained. The number of words that can be expected within a set length of time may depend on the user. For example, a lower number of words per set period of time may be expected for a user that belongs to a population that has severe speech motor impairment and/or severe cognitive impairment, compared to a user that belongs to a population that has mild speech motor impairment and/or severe cognitive impairment. The expected number of words per set period of time may be determined using a comparative training cohort. Preferably, a comparative training cohort is made up of individuals that have a similar condition, disease or disorder to the intended user, and/or a similar level of cognitive and/or speech motor impairment to the intended user. The set length of time is advantageously under 120 seconds. Tests that are too long may be influenced by external parameters such as boredom or physical weakness and/or may be less convenient for the user potentially leading to decreased uptake. A set length of time may be chosen from: 30 seconds, 35 seconds, 40 seconds, 45 seconds, 50 seconds, 55 seconds, or 60 seconds. A set length of time may advantageously be chosen based on the existence of a standard and/or comparative test. For example, a set length of time of 45 seconds is standard for the words reading part of the Stroop test as implemented in the Unified Huntington's disease rating scale (UHDRS) (as described in reference [2]).

In embodiments, a set number of words may be used instead of a set length of time. In other words, the recording may be as long as needed for the subject to read the set of words that are displayed to them. In such embodiments, the computing device may not record a voice recording of a set length of time and may instead record a voice recording until the subject indicates stops the recording, e.g. when they have read the set of words displayed. Alternatively, the computing device may record a voice recording until it detects that the subject has not spoken a word for a predetermined length of time. In other words, the method may comprise causing the computing device associated with the subject to record a voice recording from when the computing device receives a start signal to when the computing device receives a stop signal. The start and/or stop signal may be received from the subject through a user interface. Alternatively, the start and/or stop signal may be automatically generated. For example, the start signal may be generated by the computing device starting to display words. The stop signal may be generated for example by the computing device determining that the no voice signal has been detected for a set minimum period of time such as e.g. 2, 5, 10 or 20 seconds. Without wishing to be bound by theory, it is believed that the use of voice recordings that are expected to contain a known number of words (corresponding to the number of words in the set of words) may be particularly advantageous in any aspect of the invention. Indeed, such embodiments may advantageously simplify the alignment step since the known sequence of words would then have a known length for any recording.

In embodiments, the method comprises causing the causing the computing device associated with the subject to record two or more voice recordings of a set length of time. In embodiments, multiple separate voice recordings from a subject may be obtained and analysed together. This may be advantageous for example when the subject is too weak to perform a word reading task for a long set period of time. In such cases, two shorter separate voice recordings (such as e.g. two 30 or 40 seconds recordings, or two 30 or 40 words recordings) may be advantageously used instead of a single longer voice recording (such as e.g. a single 45 or 60 seconds recording, or a single 60-80 words recordings). In embodiments using multiple separate voice recordings, the steps of identifying segments corresponding to single words/syllables are advantageously performed at least in part separately for the separate voice recordings. For example, steps comprising normalisation, dynamic thresholding, scaling, etc. are advantageously performed separately for each recording. In embodiments using multiple separate voice recordings, the alignment step may be performed separately for each recording. By contrast, the clustering step may advantageously be performed on the combined data from the multiple recordings.

In other embodiments, obtaining a voice recording comprises recording a voice recording and optionally displaying a set of words to the subject prior to recording the voice recording. In such embodiments, the analysis of the voice recording may be performed by the same computing device (i.e. locally). This may advantageously remove the need for a connection to a remote device for analysis, and the need to transfer sensitive information. The results of the analysis (e.g. correct word count, speech rate etc.) and the voice recording or a compressed version thereof may in such embodiments still be communicated to a remote computing device for storage and/or meta-analysis.

In embodiments of any aspect, obtaining a voice recording comprises assessing the quality of the voice recording by determining the noise level and/or the signal-to-noise ratio of the recording. In embodiments, the signal (resp., noise) in the recording may be estimated based (such as e.g. by taking the average of) on the relative energy values assumed to correspond to signal (resp. noise), as explained above. The relative energy values assumed to correspond to signal may be e.g. the top x (where x can be e.g. 10%) relative energy values observed in the recording. Similarly, the relative energy values assumed to correspond to background noise may be e.g. the bottom x (where x can be e.g. 10%) relative energy values observed in the recording. Advantageously, where a relative energy is used, a value for the signal and/or noise in decibels can be obtained as 10*log₁₀(reIE), where relE is a relative energy value, such as the average relative energy value of the top 10% or bottom 10% of the relative energy values observed in a recording. As explained above, relative energy values may be obtained by normalising the observed power (also referred to as energy) values against the highest value observed in the recording. This leads to the highest observed energy having a relative energy of 0 dB. In such embodiments, a signal to noise ratio may be determined as the ratio of the signal estimated as explained above (e.g. average relE for top x% of observed relE in a recording) to the noise as explained above (e.g. average relE for top x% of observed relE in a recording). This can be provided as a value in dB by taking the log₁₀ of this ratio and multiplying the result by 10.

In some such embodiments, the method may comprise analysing the voice recording if the noise level is below a predetermined threshold and/or the signal level is above a predetermined threshold and/or the signal-to-noise ratio is above a predetermined threshold. A suitable threshold for noise level may be chosen as -70 dB, -60 dB, -50 dB, or -40 dB (preferably about -50 dB). A suitable threshold for signal-to-noise ratio may be chosen as 25 dB, 30 dB, 35 dB, or 40 dB (preferably above 30 dB).

In embodiments, obtaining a voice recording comprises applying one or more pre-processing procedures to a previously acquired voice recording audio file. Within the context of the present invention, a "pre-processing procedure" refers to any step applied to the voice recording data prior to analysis according to the present invention (i.e. prior to identifying single word segments).

In embodiments, obtaining a voice recording comprises applying one or more pre-processing procedures to reduce the size of a previously acquired voice recording audio file. For example, down-sampling may be used to reduce the size of the audio file used. The present inventors have found that voice recording audio files could be down-sampled to 16Hz without loss of performance of the method. This may be particularly advantageous where the analysis is performed on a remote computing device and the recording obtained at a user computing device, as it facilitates that transmission of the voice recording from the user computing device to the remote computing device.

In embodiments, assessing speech motor impairment or assessing the severity of a disease, disorder or condition in a subject comprises predicting a UHDRS dysarthria score for the subject by: defining a plurality of UHDRS dysarthria score classes corresponding to non-overlapping ranges of the UHDRS dysarthria scale; determining the speech rate associated with the voice recording from the subject; and classifying the subject as belonging to one of the plurality of UHDRS dysarthria score classes based on the determined value of the speech rate.

The Unified Huntington's Disease Rating Scale (UHDRS) is described in reference [2]. The motor assessment part of the UHDRS has a dysarthria part that provides a 5 level scale where 0=normal, 1=unclear, no need to repeat, 2=must repeat to be understood, 3=mostly incomprehensible, 4=mute.

The present inventors have found that the speech rate determined according to the present invention could be used as a reliable predictor of the UHDRS dysarthria score.

In embodiments, defining a plurality of UHDRS dysarthria score classes corresponding to non-overlapping ranges of the UHDRS dysarthria scale comprises defining two classes, a first class corresponding to UHDRS dysarthria scores at or below a threshold, and a second class corresponding to UHDRS dysarthria scores at above the threshold. In embodiments, the threshold is 0. Advantageously, such embodiments allow the classification of subjects as "normal" (first class), or showing some signs of dysarthria (second class).

In some such embodiments, classifying the subject as belonging to one of the plurality of UHDRS dysarthria score classes based on the determined value of the speech rate comprises classifying the subject as belonging to the first class if the determined speech rate for the subject is above a threshold, and in the second class otherwise.

Assessing cognitive impairment or assessing the severity of a disease, disorder or condition in a subject may comprise predicting a UHDRS Stroop word score for the subject by: determining the correct word count associated with the voice recording from the subject; and optionally scaling the correct word count. For example, the correct word count may be scaled using the length (duration) of the voice recording (such as e.g. dividing the correct word count by the length of the voice recording).

The present inventors have found that the correct word count determined as disclosed herein tightly correlates with the UHDRS-Stroop word score. In embodiments, scaling may be advantageous to make the correct word counts obtained comparable with other corresponding correct word count scores, such as e.g. those obtained according to another method or obtained with another cohort.

This may for example account for differences in length of time of the test, test modalities that may impact the expected correct word count for a given level of cognitive impairment / disease severity, etc.

According to a further aspect, there is provided a system for assessing the severity of a disease, disorder or condition in a subject, the system comprising: at least one processor; and at least one non-transitory computer readable medium containing instructions that, when executed by the at least one processor, cause the at least one processor to perform operations comprising the operations described in in relation to any of the preceding aspects.

Described herein is a system for assessing cognitive impairment and/or speech motor impairment in a subject, the system comprising: at least one processor; and at least one non-transitory computer readable medium containing instructions that, when executed by the at least one processor, cause the at least one processor to perform operations comprising: obtaining a voice recording from a word-reading test from the subject; and analysing the voice recording, or a portion thereof. Analysing the voice recording comprises: identifying a plurality of segments of the voice recording that correspond to single words or syllables; and (a) determining the speech rate associated with the voice recording by counting the number of segments identified in the voice recording; and/or (b) determining the number of correctly read words in the voice recording. Where "analysing" comprises determining the speech rate associated with the voice recording by counting the number of segments identified in the voice recording, identifying segments of the voice recording that correspond to single words or syllables comprises: obtaining a power Mel-spectrogram of the voice recording; computing the maximum intensity projection of the Mel spectrogram along the frequency axis; and defining a segment boundary as the time point where the maximum intensity projection of the Mel spectrogram along the frequency axis crosses a threshold. Where "analysing" comprises determining the number of correctly read words in the voice recording, the voice recording is from a word-reading test comprising reading a sequence of words drawn from a set of *n* words, and the method comprises: computing one or more Mel-frequency cepstral coefficients (MFCCs) for each the segments to obtain a plurality of vectors of values, each vector being associated with a segment; clustering the plurality of vector of values into *n* clusters, wherein each cluster has *n* possible labels corresponding to each of the n words; for each of the *n!* permutations of labels, predicting a sequence of words in the voice recording using the labels associated with the clustered vectors of values, and performing a sequence alignment between the predicted sequence of words and the sequence of words used in the word reading test; and selecting the labels that result in the best alignment and counting the number of matches in the alignment, wherein the number of matches corresponds to the number of correctly read words in the voice recording.

In embodiments, the instructions, when executed by the at least one processor, further cause the processor to perform the operations described in relation to any of the embodiments of the previous aspects.

Described herein is a system for analysing a voice recording from a word reading test obtained from the subject, the system comprising: at least one processor; and at least one non-transitory computer readable medium containing instructions that, when executed by the at least one processor, cause the at least one processor to perform operations comprising: identifying a plurality of segments of the voice recording that correspond to single words or syllables; and determining the speech rate associated with the voice recording by counting the number of segments identified in the voice recording. Identifying segments of the voice recording that correspond to single words or syllables comprises: obtaining a power Mel-spectrogram of the voice recording; computing the maximum intensity projection of the Mel spectrogram along the frequency axis; and defining a segment boundary as the time point where the maximum intensity projection of the Mel spectrogram along the frequency axis crosses a threshold.

In embodiments, the instructions, when executed by the at least one processor, further cause the processor to perform the operations described in relation to any of the embodiments of the previous aspects.

Described herein is a system for analysing a voice recording from a word reading test obtained from the subject, the system comprising: at least one processor; and at least one non-transitory computer readable medium containing instructions that, when executed by the at least one processor, cause the at least one processor to perform operations comprising: identifying a plurality of segments of the voice recording that correspond to single words or syllables; and determining the number of correctly read words in the voice recording, wherein the voice recording is from a word-reading test comprising reading a sequence of words drawn from a set of n words. Determining the number of correctly read words in the voice recording: computing one or more Mel-frequency cepstral coefficients (MFCCs) for the segments, to obtain a plurality of vectors of values, each vector being associated with a segment; clustering the plurality of vector of values into n clusters, wherein each cluster has n possible labels corresponding to each of the n words; for each of the n! permutations of labels, predicting a sequence of words in the voice recording using the labels associated with the clustered vectors of values, and performing a sequence alignment between the predicted sequence of words and the sequence of words used in the word reading test; and selecting the labels that result in the best alignment and counting the number of matches in the alignment, wherein the number of matches corresponds to the number of correctly read words in the voice recording. In embodiments, the instructions, when executed by the at least one processor, further cause the processor to perform the operations described in relation to any of the embodiments of the previous aspects.

Described herein is a method of diagnosing a subject as having a cognitive or speech motor impairment, the method comprising analysing a voice recording from a word-reading test from the subject, or a portion thereof, using the methods described in relation to the first aspect.

In some embodiments, the subject is diagnosed as having a cognitive or speech motor impairment if the determined speech rate and/or correct word count is lower than a threshold. An appropriate threshold may be determined for example based on a healthy reference population.

Described herein is a method of diagnosing a subject as having a disease, disorder or condition that affects speech motor and/or cognitive abilities, the method comprising analysing a voice recording from a word-reading test from the subject, or a portion thereof, using the methods described in relation to the first aspect.

In some embodiments, the subject is diagnosed as having disease, disorder or condition that affects speech motor and/or cognitive abilities if the determined speech rate and/or correct word count is lower than a threshold. An appropriate threshold may be determined for example based on a healthy reference population.

In some embodiments, the subject is diagnosed as having a cognitive or speech motor impairment if the determined speech rate and/or correct word count is lower than a threshold. An appropriate threshold may be determined for example based on a healthy reference population.

Described herein is a method of monitoring the cognitive function impairment and/or speech motor function impairment of a subject, the method comprising analysing a first voice recording obtained from the subject as described herein, analysing a second, subsequent voice recording obtained from the subject as described herein, and comparing the speech rate and/or correct word count determined by analysing the first and second voice recordings. Such embodiments may be particularly useful in the context of clinical trials.

In embodiments, the disease, disorder or condition is a neurological disease. In some embodiments, the disease is HD.

In embodiments, the disease, disorder or condition is heart failure. In some such embodiments, the speech rate and/or correct number count determined as described herein are indicative of the severity of the subject's heart failure.. In some such embodiments, the speech rate and/or correct number count determined as described herein are indicative of the severity of the subject's shortness of breath and/or level of tiredness.

In embodiments of any aspect, obtaining a voice recording from a word-reading test obtained from a subject comprises receiving a previously acquired voice recording.

Described herein is a non-transitory computer readable medium for analysing a voice recording from a word-reading test, comprising instructions that, when executed by at least one processor, cause the at least one processor to perform operations comprising: identifying a plurality of segments of the voice recording that correspond to single words or syllables; and (a) determining the number of correctly read words in the voice recording; and/or (b) determining the speech rate associated with the voice recording by counting the number of segments identified in the voice recording. Where the operations comprise determining the number of correctly read words in the voice recording, the voice recording is from a word-reading test comprising reading a sequence of words drawn from a set of *n* words, and the instructions cause the at least one processor to perform operations comprising: computing one or more Mel-frequency cepstral coefficients (MFCCs) for each the segments to obtain a plurality of vectors of values, each vector being associated with a segment; clustering the plurality of vector of values into n clusters, wherein each cluster has n possible labels corresponding to each of the n words; for each of the n! permutations of labels, predicting a sequence of words in the voice recording using the labels associated with the clustered vectors of values, and performing a sequence alignment between the predicted sequence of words and the sequence of words used in the word reading test; and selecting the labels that result in the best alignment and counting the number of matches in the alignment, wherein the number of matches corresponds to the number of correctly read words in the voice recording. Where the operations comprise determining the speech rate associated with the voice recording by counting the number of segments identified in the voice recording, identifying segments of the voice recording that correspond to single words or syllables comprises the instructions causing the at least one processor to perform operations comprising: obtaining a power Mel-spectrogram of the voice recording; computing the maximum intensity projection of the Mel spectrogram along the frequency axis; and defining a segment boundary as the time point where the maximum intensity projection of the Mel spectrogram along the frequency axis crosses a threshold.

In embodiments, the instructions, when executed by the at least one processor, further cause the processor to perform the operations described in relation to any of the embodiments of the previous aspects.

According to a further aspect, there is provided a non-transitory computer readable medium for assessing the severity of a disease, condition or disorder in a subject, comprising instructions that, when executed by at least one processor, cause the at least one processor to perform operations comprising: obtaining a voice recording from a word-reading test from the subject; and analysing the voice recording, or a portion thereof, by: identifying a plurality of segments of the voice recording that correspond to single words or syllables; and determining the number of correctly read words in the voice recording,. Where analysing the voice recording, or a portion thereof, comprises determining the number of correctly read words in the voice recording, the voice recording is from a word-reading test comprising reading a sequence of words drawn from a set of n words, and determining the number of correctly read words in the voice recording comprises: computing one or more Mel-frequency cepstral coefficients (MFCCs) for each the segments to obtain a plurality of vectors of values, each vector being associated with a segment; clustering the plurality of vector of values into *n* clusters, wherein each cluster has *n* possible labels corresponding to each of the *n* words; for each of the *n!* permutations of labels, predicting a sequence of words in the voice recording using the labels associated with the clustered vectors of values, and performing a sequence alignment between the predicted sequence of words and the sequence of words used in the word reading test; selecting the labels that result in the best alignment and counting the number of matches in the alignment, wherein the number of matches corresponds to the number of correctly read words in the voice recording, wherein the number of correctly read words is indicative of the level of cognitive impairment of the subject. Analysing the voice recording, or a portion thereof, may comprise determining the speech rate associated with the voice recording by counting the number of segments identified in the voice recording, identifying segments of the voice recording that correspond to single words or syllables comprises: obtaining a power Mel-spectrogram of the voice recording; computing the maximum intensity projection of the Mel spectrogram along the frequency axis; and defining a segment boundary as the time point where the maximum intensity projection of the Mel spectrogram along the frequency axis crosses a threshold.

In embodiments, the instructions, when executed by the at least one processor, further cause the processor to perform the operations described in relation to any of the previous aspects.

Described herein is a method of treating a neurological disorder in a subject, the method comprising analysing a voice recording from a word-reading test from the subject, or a portion thereof, using the method of any of the embodiments of the first aspect.

In embodiments, the method further comprises adapting the subject's course of treatment depending at least in part on the determined speech rate and/or correct word count.

In embodiments, adapting the subject's course of treatment comprises maintaining the subject's course of treatment if the determined speech rate and/or correct word count is not substantially lower than a previously determined speech rate and/or correct word count.

In embodiments, adapting the subject's course of treatment comprises modifying the subject's course of treatment if the determined speech rate and/or correct word count is substantially lower than a previously determined speech rate and/or correct word count.

Described herein isa method of treating a heart condition in a subject, the method comprising analysing a voice recording from a word-reading test from the subject, or a portion thereof, using the method of any of the embodiments of the first aspect.

In embodiments, the method further comprises adapting the subject's course of treatment depending at least in part on the determined speech rate and/or correct word count.

In embodiments, adapting the subject's course of treatment comprises maintaining the subject's course of treatment if the determined speech rate and/or correct word count is not substantially lower than a previously determined speech rate and/or correct word count.

In embodiments, adapting the subject's course of treatment comprises modifying the subject's course of treatment if the determined speech rate and/or correct word count is substantially lower than a previously determined speech rate and/or correct word count.

### Brief Description of the Figures

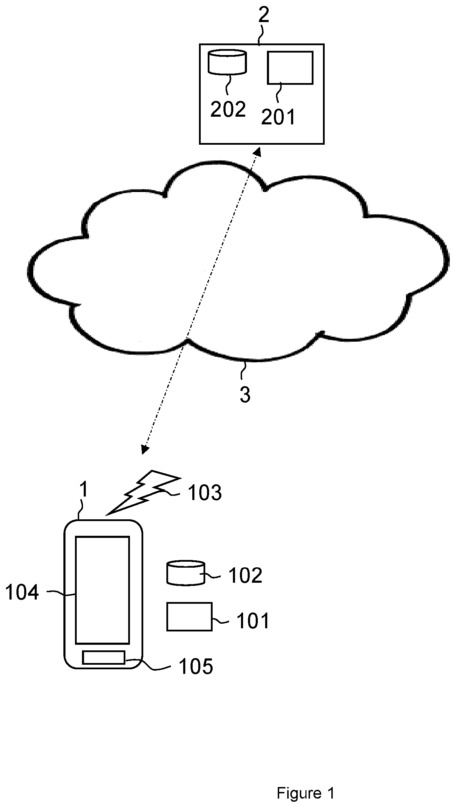

**Figure 1** shows an exemplary computing system in which embodiments of the present invention may be used.

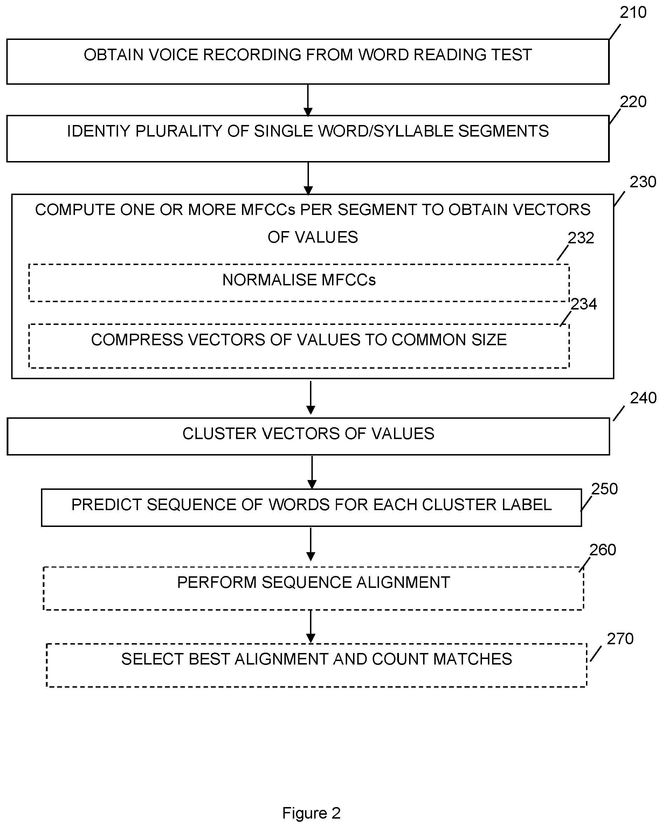

**Figure 2** is a flow chart illustrating a method of assessing cognitive impairment in a subject.

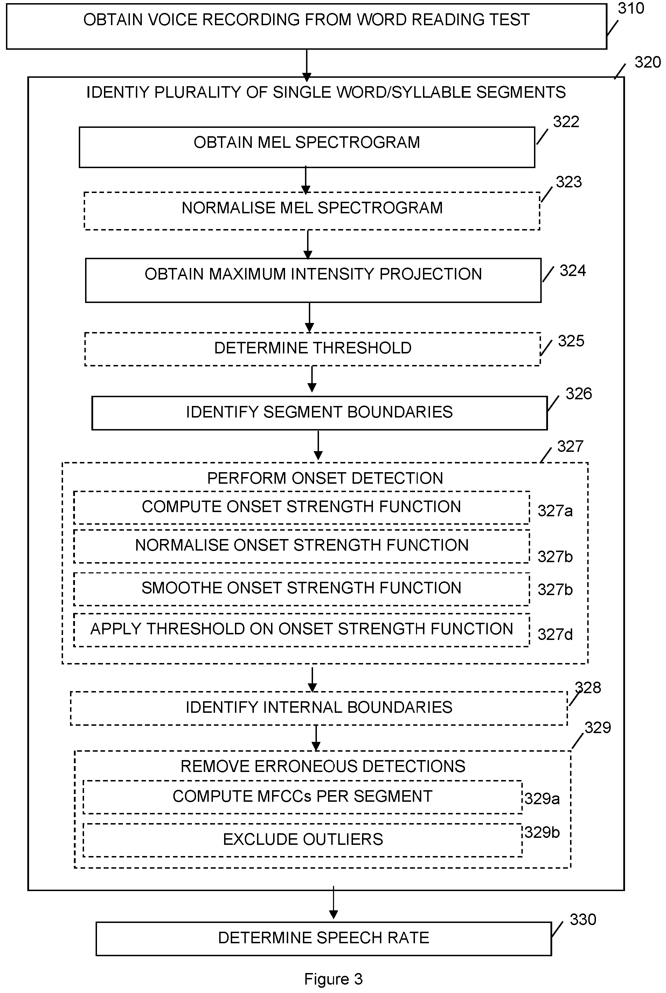

**Figure 3** is a flow chart illustrating a method of assessing speech motor impairment in a subject.

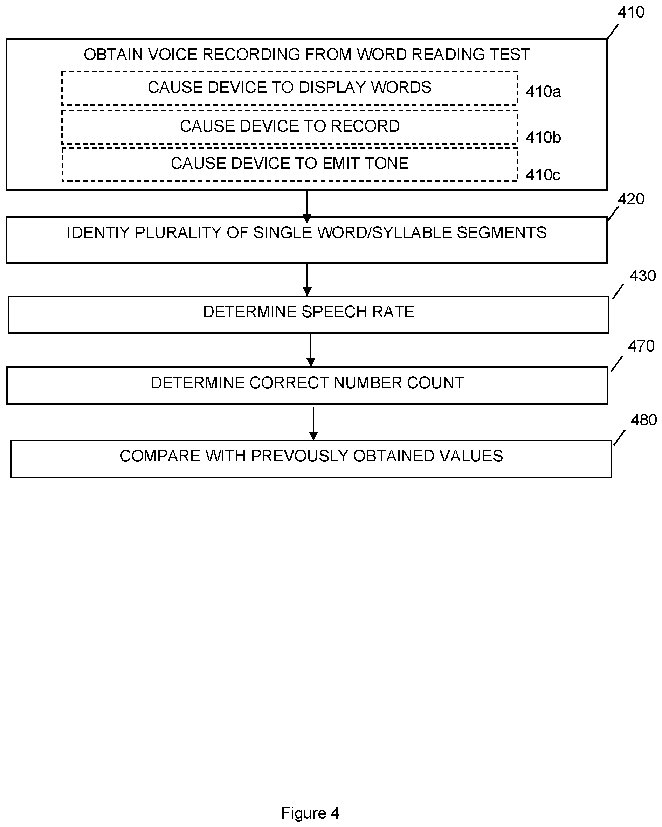

**Figure 4** illustrates schematically a method of assessing the severity of a disease, disorder or condition in a subject.

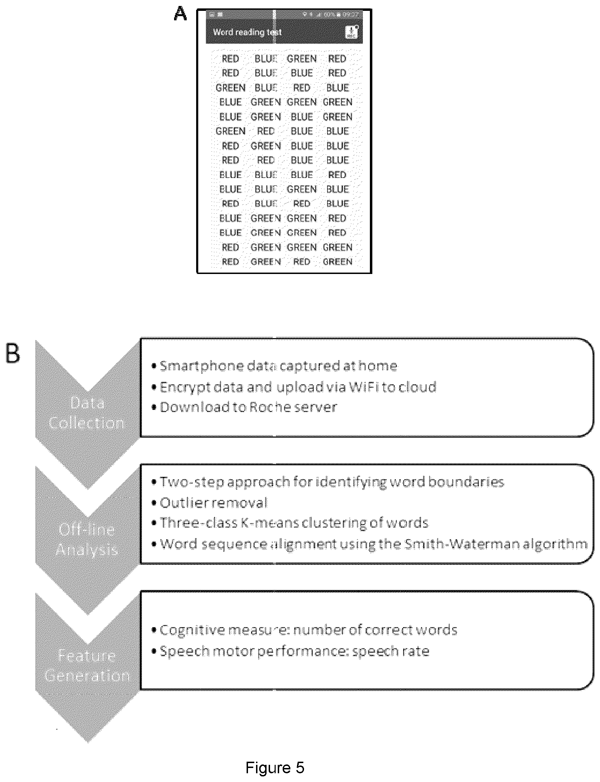

**Figures 5A and 5B** show a screenshot of a mobile device word-reading application (A) and a workflow for remote assessment (B) using the recordings from the application in (A). (A) A smartphone-based Stroop word-reading test was developed as a custom Android application. Color words were displayed in black on the screen according to a randomly generated sequence: 4 words per row and total 60 words were displayed. (B) A flow diagram describes the key steps for remote monitoring symptoms.

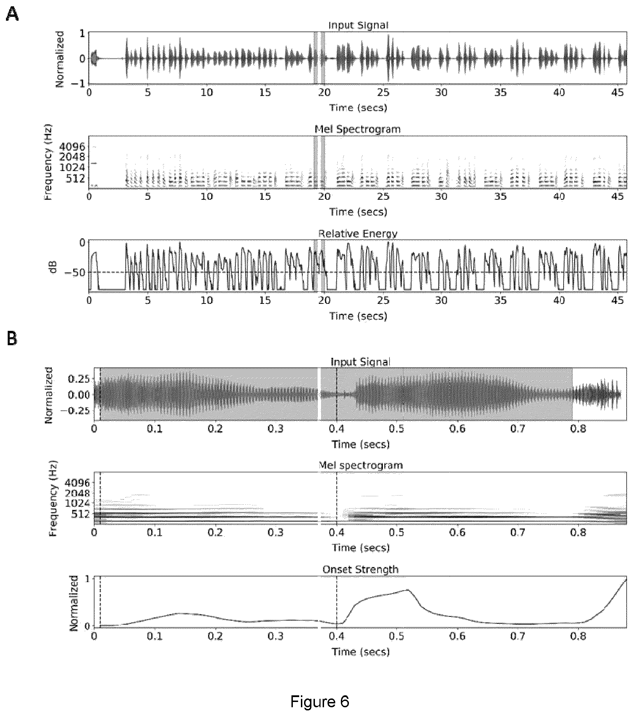

**Figures 6A and 6B** illustrate a two-step approach for identifying word boundaries according to an exemplary embodiment. (A) Coarse word boundaries were identified on the relative energy measure. A Mel-frequency spectrogram of the input audio input was constructed and the maximum intensity projection of the Mel spectrogram along frequency axis gave rise to the relative energy (B) One coarsely segmented word (highlighted in grey) was divided into two putative words based on the onset strength.

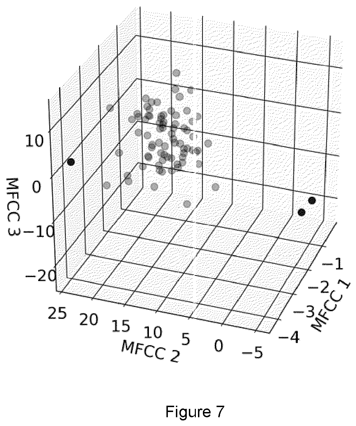

**Figure 7** illustrates an outlier removal approach according to an exemplary embodiment. All segmented words were parameterized using the first three MFCC (Mel-frequency cepstral coefficients), where inliers (putative words, n = 75) shown in grey and outliers (non-speech sounds, n = 3) in black were illustrated in a 3-D scatter plot.

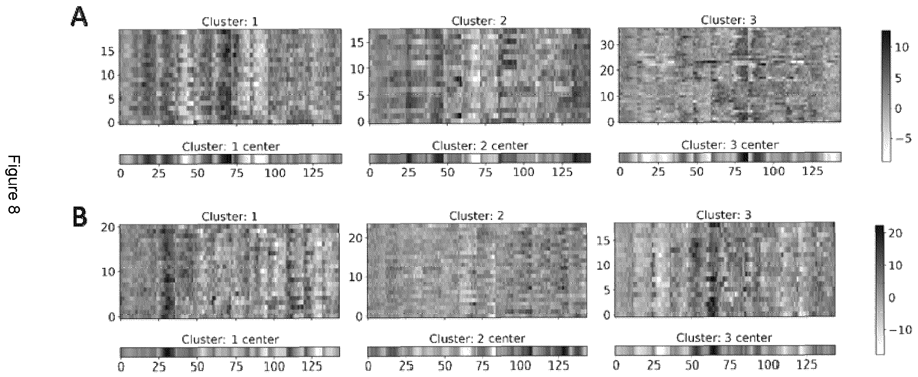

**Figures 8A and 8B** illustrates a clustering approach to identify words according to an exemplary embodiment. Putative words from one recording (where 3 different words were shown in the word reading test) were grouped into three different clusters by applying K-means clustering. Visual appearance of words in three distinctive clusters was shown in the upper graphs (one word per row) and the corresponding cluster centers were shown in the lower graphs. In particular, (A) represents 3 word-clusters from one test spoken in English (words = 75) and (B) represents 3 word-clusters from another test spoken in German (words = 64).

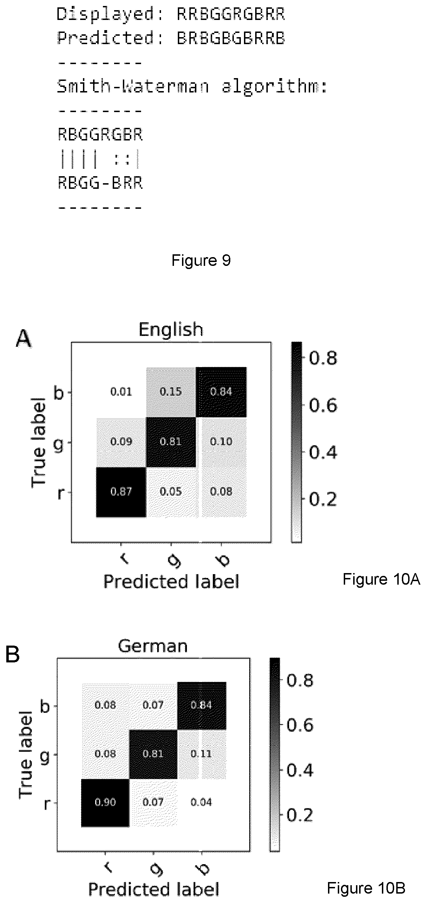

**Figure 9** illustrates a word sequence alignment approach according to an exemplary embodiment. In particular, the application of the Smith-Waterman algorithm on a 10 words sequence is shown. Alignment of displayed sequence RRBGGRGBRR and predicted sequence BRBGBGBRRB found the partially overlapping sequence and resulted in 5 correct words: matches (|), gaps (-), and mismatches (:).
**Figures 10A and 10B** show the classification accuracy of the model-free word recognition algorithm according to an exemplary embodiment. Classification accuracy of each word displayed as a normalized confusion matrix (row sum = 1). Rows represent true labels from manual annotations and columns represent predicted labels from the automated algorithm. The correct predictions are on the diagonal with a black background and the incorrect predictions are with a grey background. (A) English words: /r/ for /red/ (n = 582), /g/ for /green/ (n = 581), and /b/ for /blue/ (n = 553). (B) German words: /r/ for/rot/ (n = 460), /g/ for /grün/ (n = 459), and /b/ for /blau/ (n = 429).

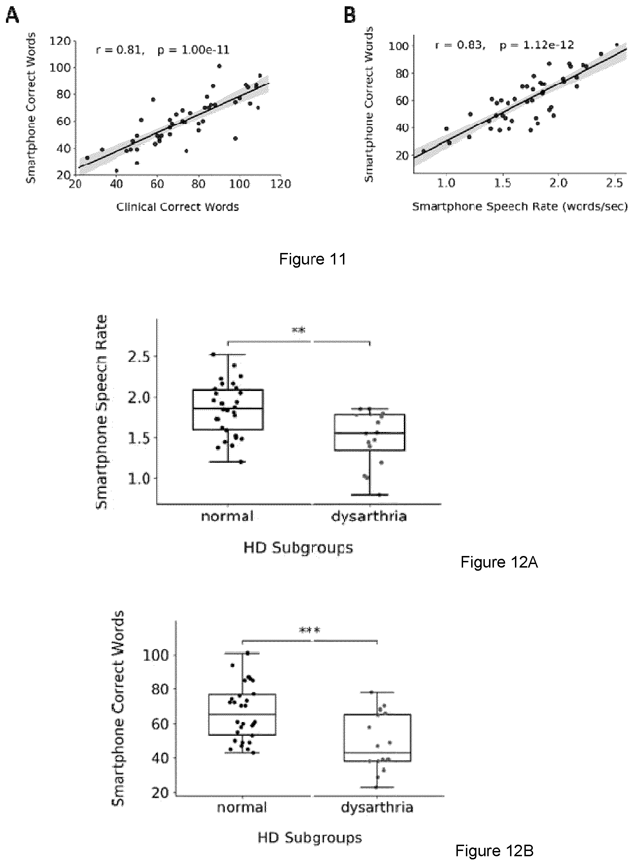

**Figures 11A and 11B** show the comparison between clinical UHDRS-Stroop word score and automated assessment measures according to an exemplary embodiment. Scatter plot of the clinical UHDRS-Stroop word score and automated assessment of the number of correct words (A) and speech rate (B) according to an exemplary embodiment are shown. A linear relationship between variables was determined through regression. The resulting regression line (black line) and a 95% confidence interval (grey shaded area) were plotted. Pearson's correlation coefficient r and the significance level of p-value were shown on both graphs.
**Figures 12A and 12B** show boxplots of speech rate and number of correct words obtained by automated assessment according to an exemplary embodiments, demonstrating that there measures were significantly reduced in subgroup of HD patients with dysarthria. Comparison between groups (normal speech n = 30, dysarthria speech n = 16): * p < 0.05; ** p < 0.01; *** p < 0.001. (A) Speech rate was significantly reduced in subgroup of HD patients with dysarthria (words/sec; 1.8 ± 0.3 vs 1.5 ± 0.3; p < 0.01; Cohens' d = 1.086). (B) The number of correct words was significantly reduced in subgroup of HD patients with dysarthria (66.8 ± 15.9 vs 48.7 ± 16.1; p < 0.001; Cohens' d = 1.110).

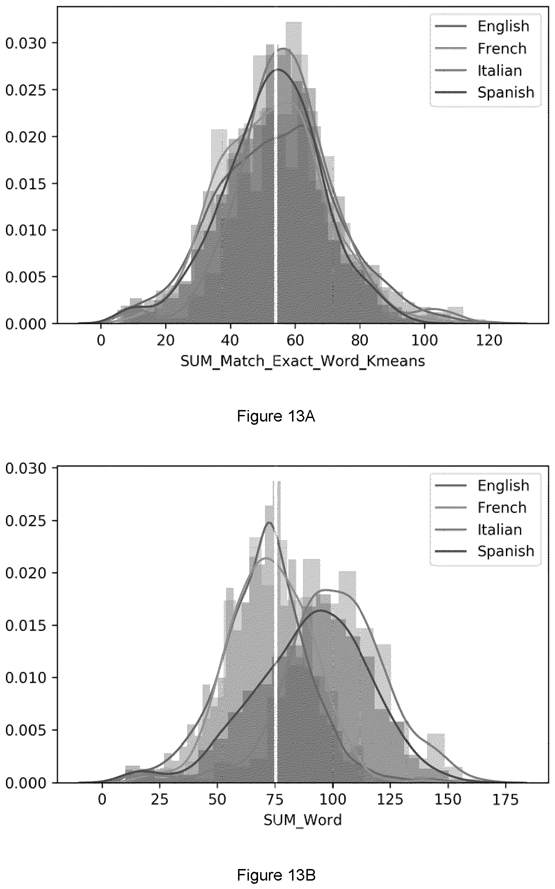

**Figures 13A and 13B** show the distribution of number of correctly read words (A) and the number of single words/syllable segments (B) identified in sets of recordings in English. French, Italian and Spanish. The data shows that the number of correctly read words identified according to the method described herein is robust to variations in the length of the words (Figure 13A), even though multiple syllables in single words are identified as separate entities (Figure 13B).

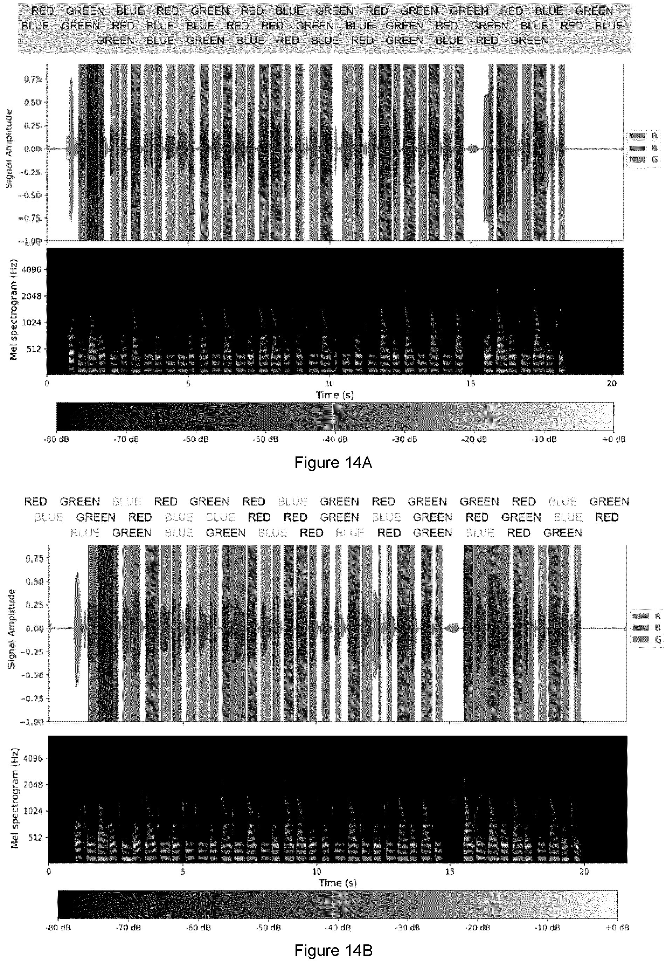

**Figures 14A and 14B** show the results of matched Stroop word reading (A, consistent condition) and Stroop colour words reading (B, interference condition) test from a healthy individual, analysed as described herein. Each subfigure shows the set of words displayed in each test (top panel), the normalised signal amplitude for the respective recording (middle panel), with overlaid segment identification and word prediction (illustrated as the colour of each segment), and the Mel spectrogram and accompanying scale (bottom panel) for the signal shown in the middle panel. The data shows that the segment identification and correct word counting processes performs equally well for both the consistent condition and the interference condition.

Where the figures laid out herein illustrate embodiments of the present invention, these should not be construed as limiting to the scope of the invention. Where appropriate, like reference numerals will be used in different figures to relate to the same structural features of the illustrated embodiments.

### Detailed Description

Specific embodiments of the invention will be described below with reference to the Figures.

**Figure 1** shows an exemplary computing system in which embodiments of the present invention may be used.

A user (not shown) is provided with a first computing device - typically a mobile computing device such as a mobile phone 1 or tablet. Alternatively, the computing device 1 may be fixed, such as e.g. a PC. The computing device 1 has at least one processor 101 and at least one memory 102 together providing at least one execution environment. Typically, a mobile device has firmware and applications run in at least one regular execution environment (REE) with an operating system such as iOS, Android or Windows. The computing device 1 may also be equipped with means 103 to communicate with other elements of computing infrastructure, for example via the public internet 3. These may comprise a wireless telecommunications apparatus for communication with a wireless telecommunications network and local wireless communication apparatus to communicate with the public internet 3 using e.g. Wi-Fi technology.

The computing device 1 comprises a user interface 104 which typically includes a display. The display 104 may be a touch screen. Other types of user interfaces may be provided, such as e.g. a speaker, keyboard, one or more buttons (not shown), etc. Further, the computing device 1 may be equipped with sound capture means, such as a microphone 105.

A second computing device 2 is also shown in Figure 1. The second computing device 2 may for example form part of an analysis provider computing system. The second computing device 2 typically comprises one or more processors 201 (e.g. servers), a plurality of switches (not shown), and one or more databases 202, and is not described further here as the details of the second computing device 2 used are not necessary for understanding how embodiments of the invention function and may be implemented. The first computing device 1 can be connected to the analysis provider computing device 2 by a network connection, such as via the public internet 3.

**Figure 2** is a flow chart illustrating a method of assessing cognitive impairment in a subject. The method comprises obtaining 210 a voice recording from a word-reading test from the subject. The voice recording is from a word-reading test comprising reading a sequence of words drawn from a (closed) set of n words.

At step 220, a plurality of segments of the voice recording that correspond to single words or syllables are identified. Step 220 may be performed as described below in relation to Figure 3 (step 320).

At steps 230-270, the number of correctly read words in the voice recording is determined. The number of correctly read words in the voice recording is indicative of the level of cognitive impairment of the subject.

In particular, at step 230, one or more Mel-frequency cepstral coefficients (MFCCs) are computed for each of the segments identified at step 220. As a result, a plurality of vectors of values is obtained, each vector being associated with a segment. In the embodiment shown on Figure 2, optional steps of normalising 232 the MFCCs across segments in the recording and compressing 234 each of the plurality of vectors to a common size are shown. In particular, a set of i MFCCs (e.g. 12 MFCCs: MFCCs 2 to 13) is computed for each frame of the segment and a set of j values (e.g. 12 values) is obtained for the segment by compressing the signal formed by each of the i MFCCs across the frames in the segment, to obtain a vector of ixj values (e.g. 144 values) for the segment.

At step 240, the plurality of vectors of values are clustered into *n* clusters (e.g. using k-means), where *n* is the expected number of different words in the word-reading test. A particular label (i.e. word identity) is not associated with each cluster. Instead, it is assumed that segments that correspond to the same word (in case of monosyllabic words) or to the same syllable of the same word (in the case of disyllabic words) will be captured by MFCCs that cluster together. In the case of disyllabic words, one of the syllables in a word may be dominant in the clustering, and it is assumed that segments corresponding to the same dominant syllable will be captured by MFCCs that cluster together. Non-dominant syllables may effectively act as noise in the clustering. Following these assumptions, each cluster should primarily group values corresponding to segments that contain one of the *n* words, and one of the *n!* possible permutation of the *n* labels for these clusters corresponds to the (unknown) true labels.

At step 250, a sequence of words in the voice recording is predicted for each of *n!* possible permutation of the *n* labels. For example, for a possible assignment of the *n* labels, a cluster is predicted for the identified segments and the corresponding label is predicted as the word that is captured in the identified segments. Some identified segments may not be associated with a cluster, for example because the MFCCs for the segment are not predicted to belong to a particular cluster with a high enough confidence. In such cases, no word may be predicted for this segment. This may be the case e.g. for segments that correspond to erroneous detections of syllables/words, or segments that correspond to a non-emphasized syllable of a multi-syllable word.

At step 260, a sequence alignment is performed (e.g. using the Smith-Waterman algorithm) between each of the predicted sequences of words and the sequence of words used in the word reading test. The sequence of words used in the word reading test may be retrieved from memory, or may be received (for example, together with the voice recording) by the processor implementing the steps of the method.

At step 270, the labels that result in the best alignment (for example, the labels that result in the highest alignment score) are selected and assumed to be the true labels for the cluster, and the number of matches in the alignment is assumed to correspond to the number of correctly read words in the voice recording .

**Figure 3** is a flow chart illustrating a method of assessing speech motor impairment in a subject. The method comprises obtaining 310 a voice recording from a word-reading test from the subject. The voice recording may be from a word-reading test comprising reading a sequence of words drawn from a (closed) set of *n* words. Any other reading test may be used. In particular, there is no requirement for the words to have any particular meaning or logical connection.

At step 320, a plurality of segments of the voice recording that correspond to single words or syllables are identified. It is particularly advantageous for the words used in the reading test to be monosyllabic as in such cases each segment may be assumed to correspond to a single word, and the timing of segments can therefore be directly related to speech rate. Where disyllabic words (or other multi-syllabic words) are used, it may be advantageous for all words to have the same number of syllables as this may simplify the calculation and/or interpretation of the speech rate.

At step 330, the speech rate associated with the voice recording is determined at least in part by counting the number of segments identified in the voice recording. The speech rate in the voice recording is indicative of the level of speech motor impairment of the subject. Optionally, determining the speech rate at step 330 may comprise computing 331 a cumulative sum of the number of identified segments in the voice recording over time, and determining 332 the slope of a linear regression model fitted to the cumulative sum data.

In particular, at step 322, a power Mel-spectrogram of the voice recording is obtained. This is typically achieved by defining frames along the voice recording (where a frame can correspond to the signal in a sliding window of fixed width applied along the time axis) and computing a power spectrum on a Mel scale for each frame (typically by obtaining a spectrogram for each frame then mapping the spectrogram to a Mel scale using overlapping triangular filters along a range of frequencies assumed to correspond to the human hearing range). This process results in a matrix of values of power per Mel unit per time bin (where a time bin corresponds to one of the positions of the sliding window). Optionally, the power Mel-spectrogram may be normalised 323, for example by dividing the values for each frame by the highest energy value observed in the recording. At step 324, the maximum intensity projection of the Mel spectrogram along the frequency axis is obtained. Segment boundaries are identified 326 as time points where the maximum intensity projection of the Mel spectrogram along the frequency axis crosses a threshold. In particular, a set of two consecutive boundaries that are such that the maximum intensity projection of the Mel spectrogram crosses the threshold from a lower to a higher value at the first boundary, and the maximum intensity projection of the Mel spectrogram crosses the threshold from a higher to a lower value a the second boundary may be considered to define a segment that corresponds to a single word or syllable. The threshold used at step 326 may optionally be dynamically determined at step 325 (where the word "dynamically determined" refers to the threshold being determined for a particular voice recording, depending on features of the particular voice recording, rather than being predetermined independently of the particular recording). For example, the threshold may be determined as a weighted average of the relative energy values assumed to correspond to signal and the relative energy values assumed to correspond to background noise.

Optionally, the segments may be "refined" by analysing separate segments identified in step 326 and determining whether further (internal) boundaries can be found. This may be performed by performing 327 onset detection for at least one of the segments by computing a spectral flux function over the Mel-spectrogram for the segment and 328 defining a further (internal) boundary whenever an onset is detected within a segment, thereby forming two new segments. Performing 327 onset detection may comprise computing 327a a spectral flux function or onset strength function, normalising 327b the onset strength function for the segment to a value between 0 and 1, smoothing 327c the (normalised) onset strength function and applying 327d a threshold to the spectral flux function or a function derived therefrom, wherein an onset is detected where the function increases above the threshold.

An optional erroneous detection removal step 329 is shown on Figure 3. In the embodiment shown, this comprises computing 329a one or more Mel-frequency cepstral coefficients (MFCCs) for the segments (preferably the first 3 MFCCs as these are expected to capture features that distinguish between noise and true utterances) to obtain a plurality of vectors of values, each vector being associated with a segment, and excluding 329b all segments whose vector of values is above a predetermined distance from the remaining vectors of values. This approach assumes that the majority of segments are correct detections (i.e. correspond to true utterances), and that segments that do not contain true utterances with have different MFCC features from correct detections. Other outlier detection methods may be applied to exclude some of the plurality of vectors of values assumed to be associated with erroneous detections.

The segments identified in step 320 may be used to determine the number of correctly read words in a word reading test as described in relation to Figure 2 (steps 230-270).

**Figure 4** illustrates schematically a method of monitoring a disease, disorder or condition in a subject. The disease, disorder or condition is one that affects speech motor and/or cognitive abilities.

The method comprises obtaining 410 a voice recording from a word-reading test from the subject. In the illustrated embodiment, obtaining a voice recording comprises causing 310a a computing device associated with the subject (e.g. computing device 1) to display a set of words (e.g. on display 104) and causing 310b the computing device 1 to record a voice recording (e.g. through microphone 105). Optionally, obtaining a voice recording may further comprises causing 310c the computing device to emit a reference tone. Obtaining 310 a voice recording from a word-reading test from the subject may instead or in addition comprise receiving a voice recording from a computing device associated with the subject (e.g. computing device 1).

The method further comprises identifying 420 a plurality of segments of the voice recording that correspond to single words or syllables. This is preferably performed as explained in relation to Figure 3. The method optionally further comprises determining 430 the speech rate associated with the voice recording, at least in part by counting the number of segments identified in the voice recording. The method further comprises determining 470 the number of correctly read words in the voice recording, as explained in relation to Figure 2 (steps 230-270). The number of correctly read words in the voice recording is indicative of the level of cognitive impairment of the subject, and the speech rate is indicative of the level of speech motor impairment of the subject. The method may further comprise comparing 480 the speech rate and correct number count obtained at steps 430 and 470 with previously obtained values for the same subject, or with one or more reference values. The comparison with previously obtained values for the same subject may be used to monitor a disease, disorder or condition in a subject who has been diagnosed as having the disease, disorder or condition. The comparison with one or more reference values may be used to diagnose the subject as having the disease, disorder or condition. For example, the reference values may correspond to a diseased population and/or a healthy population. The monitoring of a disease, disorder or condition in a subject may be used to automatically assess a course of treatment, for example as part of a clinical trial.

Any of the steps of identifying 420 a plurality of segments of the voice recording that correspond to single words or syllables, determining 430 the speech rate associated with the voice recording, and determining 470 the number of correctly read words in the voice recording may be performed by the user computing device 1, or by the analysis provider computer 2.

### Examples

### Example 1: Development of an automated smartphone-based Stroop word-reading test for the remote monitoring of disease symptoms in Huntington's disease

In this example, the inventors developed an automated smartphone-based Stroop word-reading test (SWR) and tested the feasibility of remote monitoring of disease symptoms in HD. In the smartphone-based SWR test, colour words were displayed in black on the screen according to a randomly generated sequence (4 words per row and total 60 words are displayed). Speech data were recorded with built-in microphone and uploaded via WiFi to cloud. We then developed a novel, language-independent approach to segment and classify individual words from speech signal. Finally, by comparing the displayed-word sequence with the predicted-word sequence, we were able to reliably estimate the number of correct words using the Smith-Waterman algorithm, commonly used for genomic sequence alignment.

### Methods

*Subjects and relative clinical assessments:* Forty-six patients were recruited from three sites, including Canada, Germany and the United Kingdom, as part of the HD OLE (open-label extension) study (NCT03342053). All patients underwent an extensive neurological and neuropsychological examination at the baseline visit. The Unified Huntington's Disease Rating Scale (UHDRS) was used to quantify disease severity. In particular, Stroop word-reading test (SCWT1-Word Raw Score) is part of the UHDRS cognitive assessment and dysarthria (UHDRS-dysarthria score) is part of the UHDRS motor assessment. The language spoken locally at each site was used (i.e. English in Canada and the United Kingdom n = 27, German in Germany n = 19).

*Smartphone App and self-administrated speech recordings:* A smartphone-based Stroop word-reading test was developed as a custom Android application (Galaxy S7; Samsung, Seoul, South Korea) shown in **Figure 5A** and used as part of the smart-device test suite designed for remote monitoring disease symptoms in HD by Roche. At the baseline visit, patients received a smartphone and completed a test in a teaching session. The speech tests were then performed remotely at home weekly. Speech signals were acquired at 44.1 kHz with 16-bit resolution and down sampled to 16 kHz for analysis. Data was securely transferred via WiFi to Roche, where it was processed and analysed. Data presented here were the first self-administered home tests (n = 46) only. Total 60 colour words (4 words per row) were displayed in black according to a randomly generated sequence and stored as metadata explicitly (Figure 5 A). Patients read the words after a brief reference tone (1.1 kHz, 50 ms) for a given 45-second period and restarted from the beginning once the end was reached. All recordings analysed here were with a low ambient noise level (-56.7 ± 7.4 dB, n = 46) and good signal-to-noise ratio (44.5 ± 7.8 dB, n = 46).

*Language-independent approach for analysing the Stroop word-reading test:* With consideration of potential usage in multi-language and various diseased population settings, an algorithm was designed without any pre-trained models. Words were segmented directly from the speech signal in the absence of any contextual cues. At the classification stage, word label was chosen such that it maximizes partial overlaps between displayed and predicted sequence. The fully-automated approach for the Stroop word-reading test can be divided into four parts, illustrated as a flow diagram in **Figure 5B****.** Briefly, the inventors first introduced a two-step approach to obtain a highly sensitive segmentation of individual words. The inventors then deployed an outlier removal step to filter out error detections mainly caused by imprecise articulation, respirations and non-speech sound. They then converted each putative word represented by 144 (12 x 12) Mel-frequency cepstral coefficient (MFCC) features and performed a three-class K-means clustering. Finally, the inventors adopted the Smith-Waterman algorithm, a local sequence alignment method, to estimate the number of correct words. Each of these steps will be explained in further detail below.

*Identifying word boundaries:* In this particular example, each colour word used consisted of a single syllable, i.e. /red/, /green/, /blue/ in English and /rot/, /grün/, /blau/ in German. The word segmentation therefore becomes a general syllable detection problem. According to phonology, the nucleus of a syllable also called the peak, is the central part of a syllable (most commonly a vowel), whereas consonants form the boundaries in between [9]. A number of automatic syllable detection methods have been described for connected speech [10-12]. For example, syllabic nuclei were identified mainly based upon either the wide-band energy envelope [10] or the sub-band energy envelope [11]. However, for fast speech, the transition between different syllables is difficult to identify by energy envelope alone. When considering the fast tempo and syllable repetition in the word-reading task, there is still a need for more sensitive syllable nuclei identification.

The newly developed two-step approach was motivated by how hand-label syllable boundaries were performed -visual inspection of intensity and spectral flux of a spectrogram. Briefly, a power Mel-spectrogram was first computed with a sliding window size of 15 ms and a step size of 10 ms, 138 triangular filters that span the range of 25.5 Hz to 8 kHz, and normalized against the strongest frame energy in a 45 s period. The maximal energy of a speech frame was then derived to represent intensity that is equivalent to a maximum intensity projection of the Mel-spectrogram along frequency axis. In this way, the loudest frame will have relative energy value of 0dB and others will have values below it. For example, as shown in **Figure 6A****,** all syllabic nuclei have relative energy over -50dB. Coarse word boundaries were identified by thresholding on the relative energy measure.

Subsequently, the spectral flux of the Mel-spectrogram was calculated to identify the precise boundary of each word. This is equivalent to a vertical edge detection on a Mel-spectrogram. The onset strength was computed with the superflux method developed by Böck S and Widmer G [13] and normalized to a value between 0 and 1. If the onset strength is over a threshold i.e. 0.2, the segment is divided into sub-segments. One coarsely segmented word (highlighted in grey) was divided into two putative words based on the onset strength shown in **Figure 6B****.**

All of the calculations were performed in Python, using the Librosa library (https://librosa.qithub.io/librosa/, McFee et al. [21]) or the python_speech_features library (https://qithub.com/iameslvons/pvthon speech features, James Lyons et al. [22]). For the computation of the onset strength, the function *librosa.onset.onset_strength* was used with parameters lag = 2 (time lag for computing differences) and max_size = 3 (size of the local max filter). In the example shown on Figures 6A-B, 68 coarse segments were identified in the first step, and a further 10 were identified in the refinement step.

In order to remove erroneous detections mainly caused by imprecise articulation, respirations and non-speech sound, an outlier removal step was implemented. Observations shorter than 100 ms and mean relative energy value less than -40 dB were firstly removed. Mel-frequency cepstral coefficients (MFCCs) are commonly used as features in speech recognition system [14, 15]. Here, we computed a matrix of 13 MFCCs with a sliding window size of 25 ms and a step size of 10 ms for each putative word. Audible noises are expected to differ from true words by the first three MFCCs [16]. We therefore parameterized the words using means of the first three MFCC and performed outlier detection based on the Mahalanobis distance. A cut-off value of 2 standard deviations was used to identify outliers. The inliers (putative words) shown in grey and outliers (non-speech sounds) in black were illustrated in a 3-D scatter plot in **Figure 7****.**

*K-means clustering:* K-means is an unsupervised clustering algorithm which divides observations into k clusters [17]. The inventors assumed that words pronounced by a subject at a given recording will have a similar spectral representation within word-cluster, while a different pattern between word-clusters. In this way, one can divide words into 3 clusters that is equal to the number of unique colour words. However, the duration of words may vary from one to another (mean duration between 0.23 and 0.35 ms). The steps to generate an equal sized feature representation for each word are as follows: starting from a previously computed 13 MFCCs matrix, the first MFCC (related to power) was removed from the matrix. The remaining 12 MFCCs matrix with various frame number was treated as an image and resized to a fixed-size image (12 x 12 pixels, reduced to 40%-60% of its width) by a linear interpolation along the time axis. As a result, each word was transformed to total 144 MFCC values (12 x 12 = 144) regardless of its duration. By applying K-means clustering, putative words from one recording were classified into three different clusters. **Figure 8** illustrates the visual appearance of words in three distinctive clusters shown in upper graphs (one word per row) and the corresponding cluster centres shown in lower graphs, particularly Figure 8A represents 3 word-clusters extracted from one test in English (words = 75) and Figure 8B represents 3 word-clusters extract from one test in German (words = 64).

*Word sequence alignment:* Speech recognition refers to understand the speech content. In principle, it is possible to use deep learning models (e.g. Mozilla's free speech recognition project DeepSpeech) and hidden Markov models (e.g. Carnegie Mellon University's Sphinx toolkit) to perform speech recognition. However, such pre-trained models are built on healthy population and are language dependent, and might not be very accurate when applied to patients with speech impairments. In this study, the inventors introduced a novel end-to-end solution to infer speech content. They converted such a word recognition task to a genomic sequence alignment problem. The closed-set of colour words are like the letters of the DNA code. Reading errors and system errors introduced during segmentation and clustering steps are like mutations, deletions, or insertions occurring in the DNA sequence of a gene. Instead of performing isolated word recognition, the objective was to maximize the overlapping sequence between the displayed and predicted sequence, so that the entire speech content is leveraged as a whole.

The Smith-Waterman algorithm performs the local sequence alignment that is some characters may not be considered, thus it is appropriate for partially overlapping sequences [18]. The algorithm enables to compare segments of all possible lengths and optimizes the similarity measure based on a scoring metric, e.g. a gap cost =2 match score=3. In this study, the number of segmented words defines the search space in the displayed sequence. In a three-class scenario, there are 6 (*3!*=6) possible permutations of word labels. For each permutation, it is possible to generate a predicted sequence, align with the displayed sequence, and trace back the segment that has the highest similarity score. The inventors made the assumption that subjects read words as displayed most of the time. Therefore, the segment length becomes the measure to maximize in the problem. In other words, the optimal choice of a label for a given cluster is found in a way that maximizes the overlapping sequences. Consequently, each word can be classified according to respective cluster labels. Moreover, the number of exact matches found in the partially overlapping sequences provides a good estimation of the number of correct words. **Figure 9** takes the alignment of displayed sequence RRBGGRGBRRG and predicted sequence BRBGBGBRRB as an example and returns 5 correct words out of 10 read words.

*Manual level ground truth:* Manual annotations of all segmented words (1938 words from 27 recordings in English, 1452 words from 19 recordings in German) were performed blindly via audio playback. Manual label was performed after the algorithm was designed and was not used for parameter tuning. The beginning/end time of each word was obtained by the proposed two-step approach. Words were labelled with respective text accordingly, with /r/ for /red/ and /rot/, /g/ for /green/, and /grün/ and /b/ for /blue/ and /blau/. Words that were difficult to annotate for some reasons (e.g. imprecise syllable separations, respirations, other words etc.) were labelled as /n/, as a "garbage" class.

*Outcome measures:* Based on the word segmentation and classification results, we two complementary test-level outcome measures were designed: the number of correct words for quantifying processing speed as part of the cognitive measures and the speech rate for quantifying speech motor performance. In particular, the speech rate was defined as the number of words per second and computed as the slope of the regression line on the cumulative sum of segmented words in time.

Statistical analyses: The Shapiro-Wilk test was used to test for a normal distribution. Pearson correlation was applied to examine significant relationships. The criteria used to evaluate Pearson correlation coefficient were fair (values of 0.25-0.5), moderate to good (values of 0.5-0.75) and excellent (values of 0.75 and above). ANOVA and unpaired t-test for independent samples were performed for comparison between groups. Effect sizes were measured with Cohen's d with d = 0.2 indicating a small, d = 0.5 a medium and d = 0.8 a large effect.

### Results

*Evaluation of word classification performance:* To estimate the classification accuracy of the proposed model-free word recognition algorithm, manual annotations and labels obtained by the proposed automated algorithm were compared. The overall classification accuracy was high, with an average score of 0.83 in English and 0.85 in German. The normalized confusion matrices in **Figure 10** shows the performance of our model-free word classifier at word level. The high classification accuracy suggested that the proposed word recognizer could learn all the components of a speech recognizer including the pronunciation, acoustic and language content directly from a 45-second speech recording. It leverages an unsupervised classifier and a dynamic local sequence alignment strategy to tag each word. This means, during deployment, there is no need to carry around a language model making it very practical for applications in multi-language and various diseased population settings.

*Clinical validation of two complementary outcome measures:* The number of correct words determined by the fully-automated approach was compared with the standard clinical UHDRS-Stroop word score. In general, in term of the number of correct words, the smartphone and clinical measures are highly correlated (Pearson's correlation coefficient r = 0.81, p < 0.001) shown in **Figure 11A****.**

The measures were further validated in the HD patient subgroups, who had speech impairments. Dysarthria, a corresponding clinical measure, appears as one item explicitly in the UHDRS motor evaluation section. It ranges between 0 and 4, with 1 being unclear till 4 being anarthria, unable to articulate speech at all. In the HD OLE study, there was only one patient who has dysarthria score above 1. Therefore, patients were grouped into two levels: normal speech (dysarthria score = 0, n = 30) and dysarthria speech (dysarthria score > 0, n = 16). Comparison between the normal speech subgroup and dysarthria speech subgroup showed that speech rate (words/sec; 1.8 ± 0.3 vs 1.5 ± 0.3; p < 0.01; Cohens'd = 1.086) and number of correct words (66.8 ± 15.9 vs 48.7 ± 16.1; p < 0.001; Cohens'd = 1.110) were both significantly reduced in dysarthria patients shown in **Figure 12****.** As the speech rate was directly derived from the timing of word boundaries regardless of actual labels, neither clustering nor alignment will affect the measure. In addition, the computation of the speech rate using the gradient of the regression line benefits from the entire speech. Therefore, the inventors believe that the speech rate provides a robust and sensitive measure about speech motor impairments especially in patients with dysarthria.

A strong correlation was observed between speech rate and the number of correct words (Pearson's correlation coefficient r = 0.83, p < 0.001, in **Figure 11B****)** which indicates that cognitive decline may exist side by side with speech motor impairments. Hence, extracting two complementary measures from one test is useful in understanding the interrelationships.

*Evaluation of performance in further languages:* the results obtained in this study were further expanded upon in a study including HD patients speaking 10 different languages. In particular, the methods described in this example were applied to this multi-lingual cohort using the following words: 'English': ['RED', 'GREEN', 'BLUE'], 'German': ['ROT', 'GRÜN', 'BLAU'], 'Spanish': ['ROJO', 'VERDE', 'AZUL'], 'French': ['ROUGE', 'VERT', 'BLEU'], 'Danish': ['RØD', 'GRØN', 'BLÅ'], 'Polish': ['CZERWONY','ZIELONY', 'NIEBIESKI'], 'Russian': [' 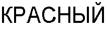 ', ' 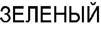 ', ' 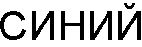 '], 'Japanese': [' 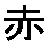 ', ' 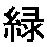 ', ' 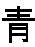 '], 'Italian': ['ROSSO', 'VERDE', 'BLU'], 'Dutch': ['ROOD', 'GROEN', 'BLAUW']. Of note, for some of these languages all of the words used were monosyllabic (e.g. English, German), whereas for **Figure 13A** shows the distribution of number of correctly read words determined from sets of recordings in English, French, Italian and Spanish, and **Figure 13B** shows the distribution of the number of segments identified (directly prior to clustering, i.e. after refinement and outlier removal) in each of these languages. The data shows that the number of correctly read words identified according to the method described above is robust to variations in the length of the words (Figure 13A), even though multiple syllables in single words are identified as separate entities (Figure 13B).

### Conclusion

In this example, the present inventors developed and showed the clinical applicability of an automated (smartphone-based) Stroop word-reading test that can be self-administered remotely from patient's home. The fully-automated approach enables to run offline analysis of speech data and allows to assess cognitive function and speech motor function in patients with HD. The approach is language-independent using an unsupervised classifier and a dynamic local sequence alignment strategy to tag each word with respect to language content. Words were classified with a high overall accuracy of 0.83 in English speaking and 0.85 in German speaking patients, without any pre-trained models. Two complementary outcome measures were clinically validated, one for assessing cognitive capability and one for evaluating speech motor impairments, in 46 patients of the HD OLE study. The number of correct words showed excellent correlation with the clinical score measured as part of the UHDRS cognitive test. A reduction of speech rate as well as worse cognitive score were pronounced in subgroup of HD patients with dysarthria speech symptoms. In summary, the approach described herein succeeded to set the ground for self-assessment of disease symptoms using smartphone based speech tests in large populations. This may ultimately bring great benefit for patients to improve quality of life for most and clinical trials to find effective treatments.

### Example 2: Automated web-based Stroop word-reading test for the remote monitoring of disease symptoms in heart failure patients

In this example, the inventors implemented the automated Stroop word-reading test (SWR) described above in the context of remote monitoring of disease symptoms in heart failure patients. The same set up as in Example 1 was used, except that the solution was deployed through a web based application, and that recordings of 40 words (and variable lengths of time) were used instead of recordings of 45 seconds. This is because many patients did not have the physical strength to perform long tests. Two recordings (i.e. 80 words in total) were combined and used for each patient, in order to ensure that the clustering step is performed using enough words to have excellent accuracy. The segment identification steps were performed separately for the two recordings, as was the alignment step. However, the clustering step was performed using the data from both recordings. The number of correct words were normalised to take into account the test duration, and the resulting normalised counts were used as the outcome measure.

Further, in addition to the consistent condition (word count), the interference part of the Stroop word-reading test was assessed as described in Example 1 (except that the words were displayed in inconsistent colours) - as described in Example 3. The inventors found that the outcome measures discussed in Example 1 (speech rate and the number of correct words, for the consistent part of the word-reading test and the interference part of the word reading test) were indicative of the patient's shortness of breath and tiredness. These could in turn be used as an indication of the patient's heart function.

### Example 3: Automated Stroop word-reading test - interference condition

In this example, the inventors tested whether the approach outlined in Example 1 could be used to automatically perform the interference part of the Stroop word-reading test. A cohort of healthy volunteers underwent both a Stroop word reading test as described in relation to Example 1, and a Stroop colour word reading test. Further, the inventors tested the performance of the method by analysing recordings for a Stroop word reading test and a Stroop colour word reading test using the same sequence of words, the words being displayed in black for the former and in inconsistent colours for the latter (see **Figures 14A and 14B****).** The results of applying the methods described in Example 1 to the two voice recordings obtained from an individual performing those matched tests are shown in Figures 14A and 14B. In these figures, segments are highlighted in the middle panel of each figure as coloured sections of signal, and the word predictions are indicated in the middle panel of each figure by the colour of the segments. The data shows that the segment identification and correct word counting processes performs equally well for both the consistent condition and the interference condition. Indeed, there is no discrepancy in cluster assignment between the word reading and interference tests, despite the presence of incorrect words read by the individual in the interference tests. Further, as can also be seen on Figure 14B, the inventors found that the predicted numbers of correctly read words obtained using the presently described automated assessment method highly correlated with the ground truth data obtained by manual annotation of the voice recordings.

### References

1. Roos, R.A., Huntington's disease: a clinical review. Orphanet J Rare Dis, 2010. 5: p. 40.
*2.* Unified Huntington's Disease Rating Scale: reliability and consistency. Huntington Study Group. Mov Disord, 1996. 11(2): p. 136-42.
3. Stroop, J.R., Studies of interference in serial verbal reactions. Journal of Experimental Psychology, 1935. General(18): p. 19.
4. Snowden, J., et al., Longitudinal evaluation of cognitive disorder in Huntington's disease. J Int Neuropsychol Soc, 2001. 7(1): p. 33-44.
5. Tabrizi, S.J., et al., Biological and clinical changes in premanifest and early stage Huntington's disease in the TRACK-HD study: the 12-month longitudinal analysis. Lancet Neurol, 2011. 10(1): p. 31-42.
6. Stout, J.C., et al., Evaluation of longitudinal 12 and 24 month cognitive outcomes in premanifest and early Huntington's disease. J Neurol Neurosurg Psychiatry, 2012. 83(7): p. 687-94.
7. Tabrizi, S.J., et al., Potential endpoints for clinical trials in premanifest and early Huntington's disease in the TRACK-HD study: analysis of 24 month observational data. Lancet Neurol, 2012. 11(1): p. 42-53.
8. Toh, E.A., et al., Comparison of cognitive and UHDRS measures in monitoring disease progression in Huntington's disease: a 12-month longitudinal study. Transl Neurodegener, 2014. 3: p. 15.
9. Kenneth, D.J., Temporal constraints and characterising syllable structuring. Phonetic Interpretation: Papers in Laboratory Phonology VI., 2003: p. 253-268.
10. Xie, Z.M. and P. Niyogi, Robust Acoustic-Based Syllable Detection. Interspeech 2006 and 9th International Conference on Spoken Language Processing, Vols 1-5, 2006: p. 1571-1574.
11. Wang, D. and S.S. Narayanan, Robust speech rate estimation for spontaneous speech. leee Transactions on Audio Speech and Language Processing, 2007. 15(8): p. 2190-2201.
12. Rusz, J., et al., Quantitative assessment of motor speech abnormalities in idiopathic rapid eye movement sleep behaviour disorder. Sleep Med, 2016. 19: p. 141-7.
13. Böck, S. and G. Widmer, Maximum filter vibrato suppression for onset detection. 16th International Conference on Digital Audio Effects, Maynooth, Ireland, 2013.
14. Davis, S.B. and P. Mermelstein, Comparison of Parametric Representations for Monosyllabic Word Recognition in Continuously Spoken Sentences. leee Transactions on Acoustics Speech and Signal Processing, 1980. 28(4): p. 357-366.
15. Huang, X., A. Acero, and H. Hon, Spoken Language Processing: A guide to theory, algorithm, and system development. Prentice Hall, 2001.
16. Rusz, J., et al., Automatic Evaluation of Speech Rhythm Instability and Acceleration in Dysarthrias Associated with Basal Ganglia Dysfunction. Front Bioeng Biotechnol, 2015. 3: p. 104.
17. Lloyd, S.P., Least-Squares Quantization in Pcm. leee Transactions on Information Theory, 1982. 28(2): p. 129-137.
18. Smith, T.F. and M.S. Waterman, Identification of common molecular subsequences. J Mol Biol, 1981. 147(1): p. 195-7.
19. Hlavnicka, J., et al., Automated analysis of connected speech reveals early biomarkers of Parkinson's disease in patients with rapid eye movement sleep behaviour disorder. Sci Rep, 2017. 7(1): p. 12.
20. Skodda, S., et al., Impaired motor speech performance in Huntington's disease. J Neural Transm (Vienna), 2014. 121(4): p. 399-407.
21. McFee, B. et al., librosa: Audio and Music Signal Analysis in Python. PROC. OF THE 14th PYTHON IN SCIENCE CONF. (SCIPY 2015).
22. James Lyons et al. (2020, January 14). jameslyons/python_speech_features: release v0.6.1 (Version 0.6.1). Zenodo. http://doi.orq/10.5281/zenodo.3607820

The terms "computer system" includes the hardware, software and data storage devices for embodying a system or carrying out a method according to the above described embodiments. For example, a computer system may comprise a central processing unit (CPU), input means, output means and data storage, which may be embodied as one or more connected computing devices. Preferably the computer system has a display or comprises a computing device that has a display to provide a visual output display (for example in the design of the business process). The data storage may comprise RAM, disk drives or other computer readable media. The computer system may include a plurality of computing devices connected by a network and able to communicate with each other over that network.

The methods of the above embodiments may be provided as computer programs or as computer program products or computer readable media carrying a computer program which is arranged, when run on a computer, to perform the method(s) described above.

The term "computer readable media" includes, without limitation, any non-transitory medium or media which can be read and accessed directly by a computer or computer system. The media can include, but are not limited to, magnetic storage media such as floppy discs, hard disc storage media and magnetic tape; optical storage media such as optical discs or CD-ROMs; electrical storage media such as memory, including RAM, ROM and flash memory; and hybrids and combinations of the above such as magnetic/optical storage media.

Unless context dictates otherwise, the descriptions and definitions of the features set out above are not limited to any particular aspect or embodiment of the invention and apply equally to all aspects and embodiments which are described.
"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

Other aspects and embodiments of the invention provide the aspects and embodiments described above with the term "comprising" replaced by the term "consisting of" or "consisting essentially of", unless the context dictates otherwise.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

## Claims

1. A method of assessing cognitive impairment in a subject, the method comprising:
obtaining (210) a voice recording from a word-reading test from the subject;
analysing the voice recording, or a portion thereof, by:
identifying (220) a plurality of segments of the voice recording that correspond to single words or syllables; and
determining the number of correctly read words in the voice recording, wherein the voice recording is from a word-reading test comprising reading a sequence of words drawn from a set of *n* words, and wherein the method comprises:
computing (230) one or more Mel-frequency cepstral coefficients (MFCCs) for the segments to obtain a plurality of vectors of values, each vector being associated with a segment;
clustering (240) the plurality of vector of values into *n* clusters, wherein each cluster has *n* possible labels corresponding to each of the *n* words;
for each of the *n!* permutations of labels, predicting (250) a sequence of words in the voice recording using the labels associated with the clustered vectors of values, and performing (260) a sequence alignment between the predicted sequence of words and the sequence of words used in the word reading test;
selecting (270) the labels that result in the best alignment and counting the number of matches in the alignment, wherein the number of matches corresponds to the number of correctly read words in the voice recording; and wherein the number of correctly read words is indicative of the level of cognitive impairment of the subject.

2. The method of claim 1, wherein the method comprises:
identifying a plurality of segments of the voice recording that correspond to single words or syllables by:
obtaining a power Mel-spectrogram of the voice recording;
computing the maximum intensity projection of the Mel spectrogram along the frequency axis; and
defining a segment boundary as the time point where the maximum intensity projection of the Mel spectrogram along the frequency axis crosses a threshold.

3. The method of claim 2, wherein identifying segments of the voice recording that correspond to single words or syllables further comprises:
normalising the power Mel-spectrogram of the voice recording, preferably against the frame that has the highest energy in the recording; and/or
performing onset detection for at least one of the segments by computing a spectral flux function over the Mel-spectrogram of the segment and
defining a further boundary whenever an onset is detected within a segment, thereby forming two new segments.

4. The method of any preceding claim, wherein the method further comprises determining the speech rate associated with the voice recording by counting the number of segments identified in the voice recording.

5. The method of claim 4, wherein determining the speech rate associated with the voice recording comprises computing a cumulative sum of the number of identified segments in the voice recording over time, and computing the slope of a linear regression model fitted to the cumulative sum data; and/or wherein the speech rate is indicative of the level of speech motor impairment of the subject.

6. The method of any preceding claim, wherein identifying segments of the voice recording that correspond to single words or syllables further comprises excluding segments that represent erroneous detections by computing one or more Mel-frequency cepstral coefficients (MFCCs) for the segments to obtain a plurality of vectors of values, each vector being associated with a segment, and applying an outlier detection method to the plurality of vectors of values.

7. The method of any preceding claim, wherein the words are colour words, optionally wherein the words are displayed in a single colour in the word reading test.

8. The method of any preceding claim, wherein computing one or more MFCCs to obtain a vector of values for a segment comprises: computing a set of *i* MFCCs for each frame of the segment for each *i* and obtaining a set of *j* values for the segment by interpolation, preferably linear interpolation, to obtain a vector of *ixj* values for the segment.

9. The method of any preceding claim, wherein clustering the plurality of vector of values into *n* clusters is performed using k-means, and/or wherein the sequence alignment step is performed using a local sequence alignment algorithm, preferably the Smith-Waterman algorithm, and/or wherein performing a sequence alignment comprises obtaining an alignment score and the best alignment is one that satisfies at least a predetermined criteria applying to the alignment score, preferably wherein the best alignment is the alignment with the highest alignment score.

10. A method of assessing the severity of a disease, disorder or condition in a subject, the method comprising analysing a voice recording from a word-reading test from the subject, or a portion thereof, as described in any of claims 1 to 9, wherein the disease, disorder or condition is one that affects speech motor and/or cognitive abilities, optionally wherein the method further comprises obtaining a voice recording from a word-reading test from the subject.

11. The method of any preceding claim, wherein obtaining a voice recording comprises receiving a word recording from a computing device associated with the subject, optionally wherein obtaining a voice recording further comprises causing a computing device associated with the subject to display a set of words and to record a voice recording.

12. The method of any of claims 4 to 11, wherein assessing speech motor impairment or assessing the severity of a disease, disorder or condition in a subject in a subject comprises predicting a UHDRS dysarthria score for the subject by:
defining a plurality of UHDRS dysarthria score classes corresponding to non-overlapping ranges of the UHDRS dysarthria scale;
determining the speech rate associated with the voice recording from the subject; and
classifying the subject as belonging to one of the plurality of UHDRS dysarthria score classes based on the determined value of the speech rate.

13. The method of any preceding claim, wherein assessing cognitive impairment or assessing the severity of a disease, disorder or condition in a subject comprises predicting a UHDRS Stroop word score for the subject by:
scaling the determined number of correctly read words in the voice recording from the subject.

14. A system for assessing the severity of a disease, condition or disorder in a subject, the system comprising:
at least one processor; and
at least one non-transitory computer readable medium containing instructions that, when executed by the at least one processor, cause the at least one processor to perform operations comprising the operations described in any of claims 1 to 13.

15. A non-transitory computer readable medium for assessing the severity of a disease, condition or disorder in a subject, comprising instructions that, when executed by at least one processor, cause the at least one processor to perform operations comprising the operations described in any of claims 1 to 13.

## Patentansprüche

1. Verfahren zum Untersuchen einer kognitiven Beeinträchtigung in einem Individuum, wobei das Verfahren Folgendes umfasst:
Erhalten (210) einer Sprachaufzeichnung von einem Wortlesetest von dem Individuum;
Analysieren der Sprachaufzeichnung oder eines Teils davon durch:
Identifizieren (220) einer Vielzahl von Segmenten der Sprachaufzeichnung, die einzelnen Wörtern oder Silben entsprechen; und
Bestimmen der Anzahl von korrekt gelesenen Wörtern in der Sprachaufzeichnung, wobei die Sprachaufzeichnung aus einem Wortlesetest stammt, der das Vorlesen einer Sequenz von Wörtern umfasst, die aus einem Satz von n Wörtern bezogen wurden, und wobei das Verfahren Folgendes umfasst:
Berechnen (230) einer oder mehrerer Mel-Frequenz-Cepstrum-Koeffizienten (MFCC) für die Segmente, um eine Vielzahl von Vektoren von Werten zu erhalten, wobei jeder Vektor einem Segment zugeordnet ist;
Clustern (240) der Vielzahl der Vektoren der Werte in n Cluster, wobei jedes Cluster n mögliche Markierungen entsprechend jedem der n Wörter aufweist;
für jede der n! Permutationen von Markierungen, Vorhersagen (250) einer Sequenz von Wörtern in der Sprachaufzeichnung unter Verwendung der mit dem geclusterten Vektoren von Werten zugeordneten Markierungen und Durchführen (260) eines Sequenzabgleichs zwischen der vorhergesagten Sequenz von Wörtern und der Sequenz von Wörtern, die in dem Wortlesetest verwendet wurde;
Auswählen (270) der Markierungen, die zum besten Abgleich führen, und Zählen der Anzahl von Übereinstimmungen in dem Abgleich, wobei die Anzahl von Übereinstimmungen der Anzahl von korrekt vorgelesenen Wörtern in der Sprachaufzeichnung entspricht; und wobei die Anzahl von korrekt vorgelesenen Wörtern das Ausmaß der kognitiven Beeinträchtigung des Individuums angibt.

2. Verfahren nach Anspruch 1, wobei das Verfahren Folgendes umfasst:
Identifizieren einer Vielzahl von Segmenten der Sprachaufzeichnung, die einzelnen Wörtern oder Silben entsprechen, durch:
Erhalten eines Leistungs-Mel-Spektrogramms der Sprachaufzeichnung;
Berechnen der Maximalintensitätsprojektion des Mel-Spektrogramms entlang der Frequenzachse; und
Definieren einer Segmentgrenze als Zeitpunkt, zu dem die Maximalintensitätsprojektion des Mel-Spektrogramms entlang der Frequenzachse einen Schwellenwert überschreitet.

3. Verfahren nach Anspruch 2, wobei das Identifizieren von Segmenten der Sprachaufzeichnung, die einzelnen Wörtern oder Silben entsprechen, ferner Folgendes umfasst:
Normalisieren des Leistungs-Mel-Spektrogramms der Sprachaufzeichnung, vorzugsweise gegenüber dem Frame, der die höchste Energie in der Aufzeichnung aufweist; und/oder
Durchführen einer Beginndetektion für zumindest eines der Segmente durch Berechnen einer Spektralflussfunktion über dem Mel-Spektrogramm des Segments und
Definieren einer weiteren Grenze, immer wenn ein Beginn innerhalb eines Segments detektiert wird, wodurch zwei neue Segmente gebildet werden.

4. Verfahren nach einem der vorangegangenen Ansprüche, wobei das Verfahren ferner das Bestimmen der Sprechrate, die der Sprachaufzeichnung zugeordnet ist, durch Zählen der Anzahl von Segmenten, die in der Sprachaufzeichnung identifiziert wurden, umfasst.

5. Verfahren nach Anspruch 4, wobei das Bestimmen der Sprechrate, die der Sprachaufzeichnung zugeordnet ist, das Berechnen einer kumulativen Summe der Anzahl von identifizierten Segmenten in der Sprachaufzeichnung über die Zeit und das Berechnen einer Steigung eines linearen Regressionsmodells, das an die kumulativen Summendaten angepasst ist, umfasst und/oder wobei die Sprechrate das Ausmaß der motorischen Sprechbeeinträchtigung des Individuums angibt.

6. Verfahren nach einem der vorangegangenen Ansprüche, wobei das Identifizieren von Segmenten der Sprachaufzeichnung, die einzelnen Wörtern oder Silben entsprechen, ferner das Ausschließen von Segmenten, die fehlerhafte Detektionen darstellen, indem ein oder mehrere Mel-Frequenz-Cepstrum-Koeffizienten (MFCC) für die Segmente berechnet werden, um eine Vielzahl von Vektoren von Werten zu erhalten, wobei jeder Vektor einem Segment zugeordnet ist, und das Anwenden eines Ausreißerdetektionsverfahrens auf die Vielzahl von Vektoren von Werten umfasst.

7. Verfahren nach einem der vorangegangenen Ansprüche, wobei die Wörter Farbwörter sind, wobei die Wörter gegebenenfalls in einer einzelnen Farbe in dem Wortlesetest angezeigt werden.

8. Verfahren nach einem der vorangegangenen Ansprüche, wobei das Berechnen eines oder mehrerer MFCC zum Erhalten eines Vektors von Werten für ein Segment Folgendes umfasst: Berechnen eines Satzes von i MFCC für jeden Frame des Segments für jedes i und Erhalten eines Satzes von j Werten für das Segment durch Interpolation, vorzugsweise lineare Interpolation, um einen Vektor von ixj Werten für das Segment zu erhalten.

9. Verfahren nach einem der vorangegangenen Ansprüche, wobei das Clustern der Vielzahl von Vektoren von Werten in n Cluster unter Verwendung von k-Mitteln durchgeführt wird und/oder wobei der Sequenzabgleichsschritt unter Verwendung eines lokalen Sequenzabgleichsalgorithmus, vorzugsweise des Smith-Waterman-Algorithmus, durchgeführt wird und/oder wobei das Durchführen eines Sequenzabgleichs das Erhalten einer Abgleichsbewertung umfasst und der beste Abgleich einer ist, der zumindest ein vorbestimmtes Kriterium erfüllt, das die Abgleichsbewertung betrifft, wobei der beste Abgleich vorzugsweise der Abgleich mit der höchsten Abgleichsbewertung ist.

10. Verfahren zur Bewertung des Schweregrads einer Erkrankung, Störung oder eines Leidens in einem Individuum, wobei das Verfahren das Analysieren einer Sprachaufzeichnung aus einem Wortlesetest von dem Individuum oder eines Teils davon, wie in einem der Ansprüche 1 bis 9 beschreiben, umfasst, wobei die Erkrankung, die Störung oder das Leiden eine(s) ist, die/das die motorischen Sprech- und/oder kognitiven Fähigkeiten beeinträchtigt, wobei das Verfahren gegebenenfalls ferner das Erhalten einer Sprachaufzeichnung aus einem Wortlesetest von dem Individuum umfasst.

11. Verfahren nach einem der vorangegangenen Ansprüche, wobei das Erhalten einer Sprachaufzeichnung das Empfangen einer Sprachaufzeichnung von einer Computervorrichtung, die dem Individuum zugeordnet ist, umfasst, wobei das Erhalten einer Sprachaufzeichnung gegebenenfalls ferner das Veranlassen umfasst, dass eine Computervorrichtung, die dem Individuum zugeordnet ist, einen Satz von Wörtern anzeigt und eine Sprachaufzeichnung aufzeichnet.

12. Verfahren nach einem der Ansprüche 4 bis 11, wobei das Bewerten einer motorischen Sprechbeeinträchtigung oder das Bewerten eines Schweregrads einer Erkrankung, Störung oder eines Leidens in einem Individuum das Vorhersagen einer UHDRS-Dysarthrie-Bewertung für das Individuum umfasst, durch:
Definieren einer Vielzahl von UHDRS-Dysarthrie-Bewertungsklassen entsprechend nichtüberlagernden Bereichen der UHDRS-Dysarthrie-Skala;
Bestimmen der Sprechrate, die der Sprachaufzeichnung von dem Individuum zugeordnet ist; und
Klassifizieren des Individuums basierend auf dem bestimmten Wert der Sprechrate als zu einer aus einer Vielzahl von UHDRS-Dysarthrie-Bewertungsklassen gehörend.

13. Verfahren nach einem der vorangegangenen Ansprüche, wobei das Bewerten der kognitiven Beeinträchtigung oder das Bewerten des Schweregrads einer Erkrankung, einer Störung oder eines Leidens in einem Individuum das Vorhersagen einer UHDRS-Stroop-Wortbewertung für das Individuum umfasst, durch:
Skalieren der bestimmten Anzahl korrekt gelesener Wörter in der Sprachaufzeichnung von dem Individuum.

14. System zum Bewerten des Schweregrads einer Erkrankung, eines Leidens oder einer Störung in einem Individuum, wobei das System Folgendes umfasst:
zumindest einen Prozessor; und
zumindest ein nichtflüchtiges computerlesbares Medium, das Befehle enthält, die, wenn sie durch den zumindest einen Prozessor ausgeführt werden, bewirken, dass der zumindest eine Prozessor Vorgänge durchführt, die Vorgänge umfassen, die in einem der Ansprüche 1 bis 13 beschrieben sind.

15. Nichtflüchtiges computerlesbares Medium zum Bewerten des Schweregrads einer Erkrankung, eines Leidens oder einer Störung in einem Individuum, umfassend Befehle, die, wenn sie durch den zumindest einen Prozessor ausgeführt werden, bewirken, dass der zumindest eine Prozessor Vorgänge durchführt, die Vorgänge umfassen, die in einem der Ansprüche 1 bis 13 beschrieben sind.

## Revendications

1. Procédé d'évaluation d'une déficience cognitive chez un sujet, le procédé comprenant les étapes consistant à :
obtenir un enregistrement vocal à partir d'un test de lecture de mots auprès du sujet ;
analyser l'enregistrement vocal, ou une partie de celui-ci, en :
identifiant (220) une pluralité de segments de l'enregistrement vocal qui correspondent à des mots ou des syllabes uniques ; et
déterminant le nombre de mots correctement lus dans l'enregistrement vocal, dans lequel l'enregistrement vocal provient d'un test de lecture de mots comprenant la lecture d'une séquence de mots tirés d'un ensemble de n mots, et dans lequel le procédé comprend les étapes consistant à :
calculer (230) un ou plusieurs coefficients cepstraux de fréquence Mel (MFCC) pour les segments afin d'obtenir une pluralité de vecteurs de valeurs, chaque vecteur étant associé à un segment ;
regrouper (240) la pluralité de vecteurs de valeurs en n groupes, dans lequel chaque groupe présente n étiquettes possibles correspondant à chacun des n mots ;
pour chacune des n ! permutations d'étiquettes, prédire (250) une séquence de mots dans l'enregistrement vocal en utilisant les étiquettes associées aux vecteurs de valeurs regroupés, et effectuer (260) un alignement de séquences entre la séquence de mots prédite et la séquence de mots utilisée dans le test de lecture de mots ;
sélectionner (270) les étiquettes qui donnent le meilleur alignement et compter le nombre de correspondances dans l'alignement, dans lequel le nombre de correspondances correspond au nombre de mots correctement lus dans l'enregistrement vocal ; et dans lequel le nombre de mots correctement lus est indicatif du niveau de déficience cognitive du sujet.

2. Procédé selon la revendication 1, dans lequel le procédé comprend les étapes consistant à :
identifier une pluralité de segments de l'enregistrement vocal qui correspondent à des mots ou des syllabes uniques en :
obtenant un spectrogramme de puissance Mel de l'enregistrement vocal ;
calculant la projection d'intensité maximale du spectrogramme Mel le long de l'axe des fréquences ; et
définissant une limite de segment comme le point temporel où la projection d'intensité maximale du spectrogramme Mel le long de l'axe des fréquences franchit un seuil.

3. Procédé selon la revendication 2, dans lequel l'identification de segments de l'enregistrement vocal qui correspondent à des mots ou des syllabes uniques comprend en outre les étapes consistant à :
normaliser le spectrogramme Mel de puissance de l'enregistrement vocal, de préférence par rapport à la trame qui présente l'énergie la plus élevée dans l'enregistrement ; et/ou
effectuer une détection de début pour au moins un des segments en calculant une fonction de flux spectral sur le spectrogramme Mel du segment, et
définir une limite supplémentaire chaque fois qu'un début est détecté dans un segment, en formant ainsi deux nouveaux segments.

4. Procédé selon l'une quelconque des revendications précédentes, dans lequel le procédé comprend en outre la détermination du débit de parole associé à l'enregistrement vocal en comptant le nombre de segments identifiés dans l'enregistrement vocal.

5. Procédé selon la revendication 4, dans lequel la détermination du débit de parole associé à l'enregistrement vocal comprend le calcul d'une somme cumulée du nombre de segments identifiés dans l'enregistrement vocal au fil du temps, et le calcul de la pente d'un modèle de régression linéaire ajusté aux données de somme cumulée ; et/ou dans lequel le débit de parole est indicatif du niveau de déficience motrice de la parole du sujet.

6. Procédé selon l'une quelconque des revendications précédentes, dans lequel l'identification de segments de l'enregistrement vocal qui correspondent à des mots ou des syllabes uniques comprend en outre l'exclusion de segments qui représentent des détections erronées en calculant un ou plusieurs coefficients cepstraux de fréquence Mel (MFCC) pour les segments afin d'obtenir une pluralité de vecteurs de valeurs, chaque vecteur étant associé à un segment, et l'application d'un procédé de détection de valeurs aberrantes à la pluralité de vecteurs de valeurs.

7. Procédé selon l'une quelconque des revendications précédentes, dans lequel les mots sont des mots en couleur, facultativement dans lequel les mots sont affichés dans une seule couleur dans le test de lecture de mots.

8. Procédé selon l'une quelconque des revendications précédentes, dans lequel le calcul d'un ou plusieurs MFCC pour obtenir un vecteur de valeurs pour un segment comprend l'étape consistant à :
calculer un ensemble de i MFCC pour chaque trame du segment pour chaque i et obtenir un ensemble de *j* valeurs pour le segment par interpolation, de préférence une interpolation linéaire, pour obtenir un vecteur de *ixj* valeurs pour le segment.

9. Procédé selon l'une quelconque des revendications précédentes, dans lequel le regroupement de la pluralité de vecteurs de valeurs en n groupes est effectué en utilisant des moyennes k, et/ou dans lequel l'étape d'alignement de séquences est effectuée en utilisant un algorithme d'alignement de séquences local, de préférence l'algorithme de Smith-Waterman, et/ou dans lequel la réalisation d'un alignement de séquences comprend l'obtention d'un score d'alignement et le meilleur alignement est celui qui satisfait au moins un critère prédéterminé s'appliquant au score d'alignement, de préférence dans lequel le meilleur alignement est l'alignement avec le score d'alignement le plus élevé.

10. Procédé d'évaluation de la gravité d'une maladie, d'un trouble ou d'un état chez un sujet, le procédé comprenant l'analyse d'un enregistrement vocal à partir d'un test de lecture de mots auprès du sujet, ou d'une partie de celui-ci, tel que décrit dans l'une quelconque des revendications 1 à 9, dans lequel la maladie, le trouble ou l'état est un état qui affecte les capacités motrices et/ou cognitives de la parole, facultativement dans lequel le procédé comprend en outre l'obtention d'un enregistrement vocal à partir d'un test de lecture de mots auprès du sujet.

11. Procédé selon l'une quelconque des revendications précédentes, dans lequel l'obtention d'un enregistrement vocal comprend la réception d'un enregistrement de mots à partir d'un dispositif informatique associé au sujet, facultativement dans lequel l'obtention d'un enregistrement vocal comprend en outre l'étape consistant à amener un dispositif informatique associé au sujet à afficher un ensemble de mots et à enregistrer un enregistrement vocal.

12. Procédé selon l'une quelconque des revendications 4 à 11, dans lequel l'évaluation d'une déficience motrice de la parole ou l'évaluation de la gravité d'une maladie, d'un trouble ou d'un état chez un sujet comprend la prédiction d'un score de dysarthrie UHDRS pour le sujet par l'intermédiaire des étapes consistant à :
définir une pluralité de classes de score de dysarthrie UHDRS correspondant à des plages non chevauchantes de l'échelle de dysarthrie UHDRS ;
déterminer le débit de parole associé à l'enregistrement vocal provenant du sujet ; et
classer le sujet comme appartenant à l'une de la pluralité de classes de score de dysarthrie UHDRS sur la base de la valeur déterminée du débit de parole.

13. Procédé selon l'une quelconque des revendications précédentes, dans lequel l'évaluation d'une déficience cognitive ou l'évaluation de la gravité d'une maladie, d'un trouble ou d'un état chez un sujet comprend la prédiction d'un score de mot UHDRS Stroop pour le sujet par l'intermédiaire de l'étape consistant à :
mettre à l'échelle le nombre déterminé de mots lus correctement dans l'enregistrement vocal provenant du sujet.

14. Système pour évaluer la gravité d'une maladie, d'un état ou d'un trouble chez un sujet, le système comprenant :
au moins un processeur ; et
au moins un support non transitoire lisible par ordinateur contenant des instructions qui, lorsqu'elles sont exécutées par le au moins un processeur, amènent le au moins un processeur à exécuter des opérations comprenant les opérations décrites dans l'une quelconque des revendications 1 à 13.

15. Support non transitoire lisible par ordinateur pour évaluer la gravité d'une maladie, d'un état ou d'un trouble chez un sujet, comprenant des instructions qui, lorsqu'elles sont exécutées par au moins un processeur, amènent le au moins un processeur à exécuter des opérations comprenant les opérations décrites dans l'une quelconque des revendications 1 à 13.
